# EUROPEAN PATENT APPLICATION

(11) **EP 3 305 902 A1**
(43) Date of publication of application: **11.04.2018**
(21) Application number: 17192852.6
(22) Date of filing: 01.03.2011
(51) Int. Cl.: C12N 15/62, C07H 21/00, C07K 19/00, C12N 15/12, C12Q 1/68

(54) **UV ASSOCIATED MTDNA FUSION TRANSCRIPTS AND METHODS AND USES THEREOF**

(30) Priority: 01.03.2010 US 309216 P
(62) Divisional of application: 11750131.2
(71) Applicant: MDNA Life Sciences Inc., Wilmington, DE 19801 (US)
(72) Inventor: HARBOTTLE, Andrew, Newcastle Upon Tyne, Tyne and Wear NE1 3DW (GB); DAKUBO, Gabriel, Thunder Bay, Ontario P7J 1H7 (CA); PARR, Ryan, Thunder Bay, Ontario P7G 1J4 (CA); CREED, Jennifer, Broomfield, CO Colorado 80023 (US); REGULY, Brian, Vancouver, British Columbia V5Z 1R3 (CA); ROBINSON, Kerry, Thunder Bay, Ontario P7A 3S9 (CA)
(74) Representative: Nederlandsch Octrooibureau

(57) **Abstract**

The present invention provides novel mitochondrial fusion transcripts and related deletion molecules that are associated with UV exposure. Methods for *in vivo* and *in vitro* detection of mtDNA molecules and associated fusion transcripts is also provided, as is their use in the screening and testing of skin care products.

## Description

### CROSS REFERENCE TO RELATED APPLICATIONS

The present invention claims priority from US Application number 61/309,216, filed March 1, 2010, the entire contents of which are incorporated herein by reference.

### FIELD OF THE INVENTION

The present invention relates to the field of mitochondrial genomics. In particular, the invention relates to mitochondrial fusion transcripts and related deletion molecules associated with UV exposure, as well as their detection and monitoring in biological samples.

### BACKGROUND OF THE INVENTION

### Mitochondrial Genome

The mitochondrial genome is a compact yet critical sequence of nucleic acids. Mitochondrial DNA, or "mtDNA", comprises a small genome of 16,569 nucleic acid base pairs (bp) (Anderson et al., 1981; Andrews et al., 1999) in contrast to the immense nuclear genome of 3.3 billion bp (haploid). The mtDNA genetic complement is substantially smaller than that of its nuclear cell mate (0.0005%). However, individual cells carry anywhere from 10³ to 10⁴ mitochondria depending on specific cellular functions (Singh and Modica-Napolitano, 2002). Communication or chemical signalling routinely occurs between the nuclear and mitochondrial genomes (Sherratt et al., 1997). Moreover, specific nuclear components are responsible for the maintenance and integrity of mitochondrial sequences (Croteau et al., 1999). All mtDNA genomes in a given individual are identical due to the clonal expansion of mitochondria within the ovum, once fertilization has occurred. However mutagenic events can induce sequence diversity reflected as somatic mutations. These mutations may accumulate in different tissues throughout the body in a condition known as heteroplasmy.

### Mitochondrial Fusion Transcriptome

The mitochondrial genome is unusual in that it is a circular, intron-less DNA molecule. The genome is interspersed with repeat motifs which flank specific lengths of sequences. Sequences between these repeats are prone to deletion under circumstances which are not well understood. Given the number of repeats in the mitochondrial genome, there are many possible deletions. The best known example is the 4977 "common deletion." This deletion has been associated with several purported conditions and diseases and is thought to increase in frequency with aging (Dai et al., 2004; Ro et al., 2003; Barron et al., 2001; Lewis et al., 2000; Muller-Hocker, 1998; Porteous et al., 1998).

Various other mitochondrial deletions have been associated with early onset of prostate, skin and lung cancer, as well as aging (e.g. Polyak et al., 1998), premature aging, and exposure to carcinogens (Lee et al., 1998). Additionally, researchers have found that ultraviolet radiation (UV) is important in the development and pathogenesis of non-melanoma skin cancer (NMSC) (Weinstock 1998; Rees, 1998) and that UV induces mtDNA damage in human skin (Birch-Machin, 2000a). In Canadian Patent Application No. 2,480,184, for example, a 3895 bp deletion in the minor arc of the mitochondrial genome was identified as a biomarker of UV-induced DNA damage. The 3895 bp deletion has also since been associated with skin cancer (PCT Application No. PCT/CA2006/000652).

As discussed in the Applicant's co-pending International Patent Application No. PCT/CA2009/000351, the knowledge gained from mapping large-scale deletions of the human mitochondrial genome has been useful in the identification of deletions associated with disease. Computer analysis of the mitochondrial genome, for example, has allowed the Applicant to identify deletion sites that, upon initiation of a deletion event in the DNA molecule, re-close or re-ligate to produce a fused DNA sequence having an open reading frame (ORF). From these studies, the Applicant identified a subset of deletion molecules and associated fusion transcripts that showed relevance to malignancy.

The Applicant has identified a further subset of mitochondrial deletions and fusion transcripts that are associated with UV exposure. Results from these investigations and their application in detecting UV damage are described herein.

### SUMMARY OF THE INVENTION

In one aspect, the present invention provides a method of detecting mitochondrial fusion transcripts.

In another aspect, the present invention provides a method of detecting a mitochondrial fusion transcript, wherein the transcript is associated with UV exposure, comprising the steps of: (a) providing a biological sample; and (b) detecting the presence of the mitochondrial fusion transcript in the sample.

In accordance with another aspect of the invention, there is provided a method for determining the cumulative UV exposure in a subject, comprising the steps of: (a) providing a biological sample from the subject; and (b) detecting the presence of a mitochondrial fusion transcript associated with UV exposure.

In accordance with another aspect of the invention, there is provided an isolated mitochondrial fusion transcript associated with UV exposure.

In accordance with another aspect of the invention, there is provided use of a fusion transcript for detecting UV exposure.

In accordance with another aspect of the invention, there is provided use of a fusion transcript as a biomarker for UV exposure.

In accordance with another aspect of the invention, there is provided a kit for detecting UV exposure, the kit comprising: (a) a probe having a sequence substantially complementary to a portion of an isolated fusion sequence of the invention.

In accordance with another aspect of the invention, there is provided a method of detecting a deletion in the human mtDNA genome, wherein said deletion is associated with UV exposure, the method comprising the steps of: (a) providing a biological sample; (b) extracting mtDNA from the biological sample; and (c)detecting the presence of a mtDNA deletion molecule.

In accordance with another aspect of the invention, there is provided a method for determining the cumulative UV exposure in a subject, comprising the steps of: (a) providing a biological sample; (b) extracting mtDNA from the biological sample; and (c) detecting the presence of a deletion in the mtDNA associate with UV exposure.

In accordance with another aspect of the invention, there is provided an isolated mtDNA deletion molecule associated with UV exposure.

In accordance with another aspect of the invention, there is provided use of a mtDNA deletion for detecting UV exposure.

In accordance with another aspect of the invention, there is provided use of a mtDNA deletion as a biomarker for UV exposure.

In accordance with another aspect of the invention, there is provided a method of testing the efficacy of a skin care product to prevent, minimize, ameliorate or protect against UV exposure or damage, the method comprising the steps of: (a) preparing a skin care product with the desired characteristics; (b) applying the skin care product to a patient's skin or a skin equivalent; (c) exposing the skin or skin equivalent to UVR; (d) detecting the presence of a mitochondrial fusion transcript associated with UV exposure in a sample of the exposed patient's skin or the exposed skin equivalent; and (e) comparing the presence of the transcript to a reference value.

In accordance with another aspect of the invention, there is provided a method of testing the efficacy of a new skin care product or formulation to prevent, minimize, ameliorate or protect against UV exposure or damage, the method comprising the steps of:
(a) preparing a skin care product with the desired characteristics; (b) applying the skin care product to a patient's skin or a skin equivalent; (c) exposing the skin or skin equivalent to UVR; (d) detecting the presence of a mtDNA deletion molecule in a sample of the exposed patient's skin or the exposed skin equivalent, wherein the mtDNA deletion molecule is associated with UV exposure; and (e) comparing the presence of the molecule to a reference value.

In accordance with another aspect of the invention, there is provided a method of screening skin care products for the ability to prevent, minimize, ameliorate or protect against UV exposure or damage, the method comprising the steps of: (a) applying the skin care products to a patient's skin or a skin equivalent; (b) exposing the skin or skin equivalent to UVR; (c) detecting the presence of a mitochondrial fusion transcript associated with UV exposure; and (e) comparing the presence of the fusion transcript for each skin care product tested against a reference value and/or each other.

In accordance with another aspect of the invention, there is provided a method of screening skin care products for the ability to prevent, minimize, ameliorate or protect against UV exposure or damage, the method comprising the steps of: (a) applying the skin care product to a patient's skin or a skin equivalent; (b) exposing the skin or skin equivalent to UVR; (c) detecting the presence of a mtDNA deletion molecule in a sample of the exposed patient's skin or the exposed skin equivalent, wherein the mtDNA deletion molecule is associated with UV exposure; and (e) comparing the presence of the deletion molecule for each skin care product tested against a reference value and/or each other.

In accordance with another aspect of the invention, there is provided a method of testing the efficacy of a skin care product to prevent, ameliorate or protect against skin aging or photo-aging, the method comprising the steps of: (a) preparing a skin care product with the desired characteristics; (b) applying the skin care product to a patient's skin or a skin equivalent; (c) exposing the skin or skin equivalent to UVR; (d) detecting the presence of a mitochondrial fusion transcript associated with UV exposure in a sample of the exposed patient's skin or the exposed skin equivalent; and (e) comparing the presence of the transcript to a reference value.

In accordance with another aspect of the invention, there is provided a method of testing the efficacy of a new skin care product or formulation to prevent, ameliorate or protect against skin aging or photo-aging, the method comprising the steps of: (a) preparing a skin care product with the desired characteristics; (b) applying the skin care product to a patient's skin or a skin equivalent; (c) exposing the skin or skin equivalent to UVR; (d) detecting the presence of a mtDNA deletion molecule in a sample of the exposed patient's skin or the exposed skin equivalent, wherein the mtDNA deletion molecule is associated with UV exposure; and (e) comparing the presence of the molecule to a reference value.

In accordance with another aspect of the invention, there is provided a method of screening skin care products for the ability to prevent, ameliorate or protect against skin aging or photo-aging, the method comprising the steps of: (a) applying the skin care products to a patient's skin or a skin equivalent; (b) exposing the skin or skin equivalent to UVR; (c) detecting the presence of a mitochondrial fusion transcript associated with UV exposure; and (e) comparing the presence of the fusion transcript for each skin care product tested against a reference value and/or each other.

In accordance with another aspect of the invention, there is provided a method of screening skin care products for the ability to prevent, ameliorate or protect against skin aging or photo-aging, the method comprising the steps of: (a) applying the skin care product to a patient's skin or a skin equivalent; (b) exposing the skin or skin equivalent to UVR; (c) detecting the presence of a mtDNA deletion molecule in a sample of the exposed patient's skin or the exposed skin equivalent, wherein the mtDNA deletion molecule is associated with UV exposure; and (e) comparing the presence of the deletion molecule for each skin care product tested against a reference value and/or each other.

### BRIEF DESCRIPTION OF THE DRAWINGS

These and other features of the invention will become more apparent in the following detailed description in which reference is made to the appended drawings.
Figure 1 shows an amino acid sequence of Frame 2 from positions 386-547:4443-5511 after removal of nucleotide positions 548-4442.
Figure 2 shows the reading frames produced after the removal of nucleotide positions 548-4442. Nucleotide 382 is denoted by a red box identifying the beginning of Reading Frame 2 in relation to position 1 of the mitochondrial genome.
Figure 3 shows the contributing sequences of the Frame 2 fusion transcript formed by the removal of nucleotide positions 548-4442.
Figure 4 shows the final amino acid sequence of the fusion transcript form by the deletion of nucleotide positions 548-4442. The sequence includes the recombined nucleotide positions 470-547 and 4443-5511 of the mitochondrial genome.
Figure 5A shows an *in vivo* UVR dose response using mtDNA deletion analysis.
Figure 5B shows a sample calculation for determining UV damage.
Figures 6-9 show the expression of fusion transcripts 2, 3, 11, 12, 20 and 32 of the present invention following UV dosing in skin equivalents.
Figures 10-12 show *in vivo* and *in vitro* testing of three UV formulations.
Figures 13 and 14 show screening of various sunscreen and anti-aging brands following UV exposure.
Figure 15 shows the UV spectrum used for the solar-simulated irradiation of Example 3.
Figure 16 shows the UV spectrum used for the solar-simulated irradiation of Example 4.

### DETAILED DESCRIPTION OF THE INVENTION

The present invention provides novel mitochondrial fusion transcripts and related mtDNA molecules that are associated with UV exposure. The invention also provides for the detection and monitoring of fusion transcripts and associated mtDNA molecules in biological samples. As well, the invention provides for the use of fusion transcripts and related mtDNA deletions in the screening and testing of skin care products.

The techniques and procedures of the invention are generally performed according to conventional methods in the art and various general references (see, for example, Sambrook et al. Molecular Cloning: A Laboratory Manual, 2d ed. (1989) Cold Spring Harbor Laboratory Press, Cold Spring Harbor, N.Y., and Lakowicz, J. R. Principles of Fluorescence Spectroscopy, New York: Plenum Press (1983)).

### Definitions

Unless defined otherwise, all technical and scientific terms used herein have the same meaning as commonly understood by one of ordinary skill in the art to which this invention belongs. The following terms, unless otherwise indicated, shall be understood to have the following meanings:

As used herein, the term "about" is intended to refer to a variation from the stated value or factor. It is to be understood that such a variation is always included in any given value or factor provided herein, whether or not it is specifically referred to. By way of example, such variation may be approximately +/-10%. It is also well known to all persons skilled in the art that there are degrees of error necessarily associated with measurement. The degree of error will vary depending on the precision of the instrument used to take the reading. Since the Applicant cannot know the precision of the instrument that will be used by persons working the invention, the degree of measurement error cannot be necessarily defined. Likewise, the accuracy of the nucleotide and amino acid sequences submitted herewith is dependent on the accuracy of the equipment and process used. Therefore, minor sequence variations are not considered to fall outside of the teaching of this application.

As used herein, the expression "mitochondrial fusion transcript" or "fusion transcript" refers to an RNA transcription product produced as a result of the transcription of a mutated mitochondrial DNA sequence wherein such mutations may comprise mitochondrial deletions and other large-scale mitochondrial DNA rearrangements.

The term "hybridize," as used herein, refers to the ability of a nucleic acid to bind detectably and specifically to a second nucleic acid. Polynucleotides, oligonucleotides and fragments thereof hybridize to direct target nucleic acid strands under hybridization and wash conditions that minimize appreciable amounts of detectable binding to non-specific nucleic acids. High stringency conditions can be used to achieve selective hybridization conditions as known in the art. Typically, hybridization and washing conditions are performed at high stringency according to conventional hybridization procedures. Washing conditions are generally 1-3 x SSC, 0.1-1% SDS, 50-70°C with a change of wash solution after about 5-30 minutes.

The term "ameliorate" includes the arrest, prevention, decrease, or improvement in one or more the symptoms, signs, or features of UV damage, both temporary and long-term.

As used herein, "aberration" or "mutation" encompasses modifications in the wild type mitochondrial DNA sequence that results in a fusion transcript and includes, without limitation, substantive or large-scale mtDNA deletions.

As used herein, "mitochondrial DNA" or "mtDNA" is DNA present in or originated in mitochondria.

The term "subject" or "patient" as used herein refers to an animal in need of treatment or an animal being tested.

The term "animal," as used herein, refers to both human and non-human animals, including, but not limited to, mammals, birds and fish.

As used herein the term "derived from," as applied to an object indicates that the object is obtained from a specified source, albeit not necessarily directly from that source.

As used herein, "diagnostic" or "diagnosing" means using the presence or absence or quantity of a mutation or combination of mutations as a factor in UV exposure diagnosis or UV exposure management.

The term "skin" refers to the outer protective covering of the body, consisting of the corium and the epidermis, and is understood to include sweat and sebaceous glands, as well as hair follicle structures. In one embodiment, the skin is mammalian skin, preferably human.

As used herein, "biological sample" refers to a tissue or bodily fluid containing cells from which a molecule of interest can be obtained. For example, the biological sample can be derived from skin cells or tissue, wherein tissue may be taken from the dermis or epidermis, or a combination of both. The biological sample can be used either directly as obtained from the source or following a pre-treatment to modify the character of the sample. The sample may be obtained by a variety of methods including, but not limited to, punch biopsy, surgical excision, and non-invasive or minimally invasive skin sampling methods such as a wet swabbing, tapelift, cotton tip swabbing, scraping of skin using a sterile surgical blade, scraping of skin using a wooden scraper, sticky surface of an adhesive pad (CapSure™ Clean-up Pad, Arcturus), film from LCM MacroCap™ (Arcturus), heated film from LCM MacroCap™ (Arcturus) and employing a small gauge needle (for example, 28 gauge), to collect micro-cores of skin tissue. These methods are well known in the art (see, for example, Applicant's co-pending applications PCT/CA2007/001790 and PCT/CA2008/001801, which disclose various methods for collecting skin samples).

### Genomic Mutations

MtDNAs are useful biomarkers in the identification of risk factors or disruptive cellular processes associated with disease onset and environmental exposure to factors such as toxins, carcinogens or harmful radiation. According to the present invention, large-scale rearrangement mutations in the mitochondrial genome result in the identification of fusion transcripts associated with UV exposure. Thus, the use of mtDNA encoding such transcripts and probes directed thereto for the detection, diagnosis and monitoring of UV exposure is provided herein.

The methodologies of the present invention are also useful in the identification of skin care products either through the detection of mtDNA deletions or their associated fusion transcripts. Thus, the present application provides for various methods to detect (and measure) the amount of UV related damage in a biological sample, as well as to screen and test for products that can reduce or prevent such damage.

One of skill in the art will appreciate that the mtDNA molecules for use in the methods of the present invention may be derived through the isolation of naturally-occurring mutants or may be based on the complementary sequence of any of the fusion transcripts described herein. Exemplary mtDNA sequences and fusion transcripts are disclosed in the Applicant's co-pending PCT application (PCT/CA2009/000351), which is incorporated herein by reference.

### Detection of Mutant Genomic Sequences

Mutant sequences associated with UV exposure according to the present invention comprise mtDNA deletions that result in the generation of a fusion transcript. While the modification or change can vary greatly in size from only a few bases to several kilobases, preferably the modification results in a substantive or large-scale mtDNA deletion, also termed genomic aberration.

Techniques for extracting and isolating aberrant mtDNA molecules have been previously disclosed in the Applicant's co-pending patent application (PCT/CA2009/000351). Such methods, which also include techniques for selecting appropriate primers, probes and genomic sequences (i.e. those having a novel mtDNA junction point), are incorporated herein by reference.

According to an aspect of the present invention, to determine candidate genomic sequences, a junction point of a sequence deletion is first identified. Sequence deletions are primarily identified by direct and indirect repetitive elements which flank the sequence to be deleted at the 5' and 3' end. The removal of a section of the nucleotides from the genome followed by the ligation of the genome results in a fused DNA sequence with an open reading frame (ORF) and novel junction point.

Exemplary mtDNA molecules for use in the methods of the present invention are provided below. As previously described (see PCT/CA2009/000351), these mtDNAs are based on modifications of the known mitochondrial genome (SEQ ID NO: 1) and have been assigned a fusion or "FUS" designation, wherein A:B represents the junction point between the last mitochondrial nucleotide of the first spliced gene and the first mitochondrial nucleotide of the second spliced gene. The identification of the spliced genes is provided in parentheses followed by the corresponding sequence identifier. Where provided below, (AltMet) and (OrigMet) refer to alternate and original translation start sites, respectively. FUS 8469:13447 (AltMet) (ATP synthase F0 subunit 8 (ATPase8) to NADH dehydrogenase subunit 5 (ND5)) (SEQ ID No: 2)
FUS 10744:14124 (NADH dehydrogenase subunit 4L (ND4L) to NADH dehydrogenase subunit 5 (ND5)) (SEQ ID No: 3)
FUS 7974:15496 (Cytochrome c oxidase subunit II (COII) to Cytochrome b (Cytb)) (SEQ ID No: 4)
FUS 7992:15730 (Cytochrome c oxidase subunit II (COM) to Cytochrome b (Cytb)) (SEQ ID No: 5)
FUS 8210:15339 (Cytochrome c oxidase subunit II (COM) to Cytochrome b (Cytb)) (SEQ ID No: 6)
FUS 8828:14896 (ATP synthase FO subunit 6 (ATPase6) to Cytochrome b (Cytb)) (SEQ ID No: 7)
FUS 10665:14856 (NADH dehydrogenase subunit 4L (ND4L) to Cytochrome b (Cytb)) (SEQ ID No: 8)
FUS 6075:13799 (Cytochrome c oxidase subunit I (COI) to NADH de hydrogenase subunit 5 (ND5)) (SEQ ID No: 9)
FUS 6325:13989 (Cytochrome c oxidase subunit I (COI) to NADH dehydrogenase subunit 5 (ND5)) (SEQ ID No: 10)
FUS 7438:13476 (Cytochrome c oxidase subunit I (COI) to NADH dehydrogenase subunit 5 (ND5)) (SEQ ID No: 11)
FUS 7775:13532 (Cytochrome c oxidase subunit II (COM) to NADH dehydrogenase subunit 5 (ND5)) (SEQ ID No: 12)
FUS 8213:13991 (Cytochrome c oxidase subunit II (COM) to NADH dehydrogenase subunit 5 (ND5)) (SEQ ID No: 13)
FUS 9144:13816 ((ATP synthase FO subunit 6 (ATPase6) to NADH dehydrogenase subunit 5 (ND5)) (SEQ ID No: 14)
FUS 9191:12909 (ATP synthase FO subunit 6 (ATPase6) to NADH dehydrogenase subunit 5 (ND5)) (SEQ ID No: 15)
FUS 9574:12972 (Cytochrome c oxidase subunit III (COIII) to NADH dehydrogenase subunit 5 (ND5)) (SEQ ID No: 16)
FUS 10367:12829 (NADH dehydrogenase subunit 3 (ND3) to NADH dehydrogenase subunit 5 (ND5)) (SEQ ID No: 17)
FUS 11232:13980 (NADH dehydrogenase subunit 4 (ND4) to NADH dehydrogenase subunit 5 (ND5)) (SEQ ID No: 18)
FUS 8469:13447 (OrigMet) (ATP synthase FO subunit 8 (ATPase 8) to NADH dehydrogenase subunit 5 (ND5)) (SEQ ID No: 19)
FUS 547:4443 (Met to NADH dehydrogenase subunit 2 (ND2)) (SEQ ID No: 20)

As described in the examples below, once the sequences are identified their association with UV exposure is analysed. Such testing may be carried out *in vivo* in a subject or patient (see Example 5), or through the testing of skin cultures that are grown and dosed with varying levels of UVR (see Examples 3 and 4). Expression of these sequences may also be monitored in respect of one or more house keeper genes including, but not limited to, Human PPIB, Human PGK-1, Human ACTB (beta actin),Human GUSB (Beta Glucuronidase), Human B2M (Beta-2-microglobulin), Human PPIA (peptidylprolyl isomerase A (cyclophilin A)), Human GAPD (glyeraldehyde-3-phosphate dehydrogenase), Human HPRT1 (hypoxanthine phosporibosyltransferase 1), Human RPLPO = LRP (ribosmal protein, large, PO), Human HMBS = PBGD (hydroxymethlbilane synthase), Human TBP (TATA box binding protein), Human TRFC (transferrin receptor (p90, CD71), and Human ABCC13 (Chromosome 13 - psuedogene).

In one embodiment of the invention, the following mtDNA sequences are determined useful for predicting, diagnosing or monitoring UV damage, and for testing and screening skin care products effective in preventing or ameliorating UV damage:
SEQ ID NO: 3 (FUS 10744:14124)
SEQ ID NO: 4 (FUS 7974:15496)
SEQ ID NO: 12 (FUS 7775:13532)
SEQ ID NO: 13 (FUS 8213:13991)
SEQ ID NO: 19 (FUS 8469:13447; OrigMet)
SEQ ID NO: 20 (FUS 547:4443).

In another embodiment of the invention, mtDNA sequences having SEQ ID NOs: 19 and 20 are preferably used in carrying out the methods of the invention.

In addition to the above sequences, the present invention also provides the use of variants or fragments of these sequences for predicting, diagnosing and/or monitoring UV damage or exposure.

"Variant", as used herein, refers to a nucleic acid differing from a mtDNA sequence of the present invention, but retaining essential properties thereof. Generally, variants are overall closely similar, and, in many regions, identical to a select mtDNA sequence. Specifically, the variants of the present invention comprise at least one of the nucleotides of the junction point of the spliced genes, and may further comprise one or more nucleotides adjacent thereto. In one embodiment of the invention, the variant sequence is at least 80%, 85%, 90%, 95%, 96%, 97%, 98% or 99% identical to any one of the mtDNA sequences of the invention, or the complementary strand thereto.

In the present invention, "fragment" refers to a short nucleic acid sequence which is a portion of that contained in the disclosed genomic sequences, or the complementary strand thereto. This portion includes at least one of the nucleotides comprising the junction point of the spliced genes, and may further comprise one or more nucleotides adjacent thereto. The fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases of any one of the mtDNA sequences listed above. In this context "about" includes the particularly recited value, a value larger or smaller by several (5, 4, 3, 2, or 1) nucleotides, at either terminus or at both termini. These fragments have uses that include, but are not limited to, as diagnostic probes and primers as discussed herein. Of course, larger fragments (e.g., 50, 150, 500, 600, 2000 nucleotides) are also contemplated.

### Primers and Probes

Another aspect of the invention is to provide a primer or probe capable of recognizing an mtDNA sequence of the invention. As used herein, the terms "primer" and "probe" refer to an oligonucleotide which forms a duplex structure with a sequence in the target nucleic acid, due to complementarity of at least one sequence in the primer or probe with a sequence in the target region. The probe may be labeled, according to methods known in the art.

Once aberrant mtDNA associated with UV exposure is identified, hybridization of mtDNA to, for example, an array of oligonucleotides can be used to identify particular mutations, however, any known method of hybridization may be used.

The preparation of suitable primers and probes for use in the present invention may be generated as described in the Applicant's co-pending PCT application. In this regard, probes can be generated directly against exemplary mtDNA fusion molecules of the invention, or to a fragment or variant thereof. For instance, the sequences set forth in SEQ ID NOs: 3, 4 12, 13, 19 and 20 can be used to design primers or probes that will detect a nucleic acid sequence comprising a fusion sequence of interest. As would be understood by those of skill in the art, primers or probes which hybridize to these nucleic acid molecules may do so under highly stringent hybridization conditions or lower stringency conditions, such conditions known to those skilled in the art and found, for example, in Current Protocols in Molecular Biology (John Wiley & Sons, New York (1989)), 6.3.1-6.3.6. Primers and probes of the invention may also hybridize to target sequences where there is DNA slippage or where a similar sequence altering event has occurred.

In specific embodiments of the invention, the probes of the invention contain a sequence complementary to at least a portion of the aberrant mtDNA comprising the junction point of the spliced genes. This portion includes at least one of the nucleotides involved in the junction point A:B, and may further comprise one or more nucleotides adjacent thereto. In this regard, the present invention encompasses any suitable targeting mechanism that will select an mtDNA molecule using the nucleotides involved and/or adjacent to the junction point A:B.

Various types of probes (as described in the Applicant's co-pending PCT application) are contemplated by the present invention. The probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

The probes of the invention will also hybridize to nucleic acid molecules in biological samples, thereby enabling the methods of the invention. Accordingly, in one aspect of the invention, there is provided a hybridization probe for use in detecting, diagnosing and/or monitoring UV damage or exposure, wherein the probe is complementary to at least a portion of an aberrant mtDNA molecule. In another aspect the present invention provides probes and a use of (or a method of using) such probes for screening or testing skin care products effective in preventing or ameliorating UV damage or exposure.

### Assays

Measuring the level of aberrant mtDNA in a biological sample can determine the level of UV exposure in a subject. The present invention, therefore, encompasses methods for detecting, diagnosing or monitoring UV damage or exposure, comprising obtaining one or more biological samples, extracting mtDNA from the samples, and assaying the samples for aberrant mtDNA by: quantifying the amount of one or more aberrant mtDNA sequences in the sample and comparing the quantity detected with a reference value.

As would be understood by those of skill in the art, the reference value is based on whether the method seeks to detect, diagnose or monitor UV damage or exposure. Accordingly, the reference value may relate to mtDNA data collected from one or more known UV exposed biological samples, from one or more known non-UV exposed biological samples, and/or from one or more biological samples taken over time. The sample may be derived from rarely sun exposed, occasionally sun exposed, usually sun exposed skin or blood and collected by a variety of methods including, but not limited to, punch biopsy, surgical excision, and non-invasive or minimally invasive skin sampling methods such as a wet swabbing, tapelift, cotton tip swabbing, scraping of skin using a sterile surgical blade, scraping of skin using a wooden scraper, sticky surface of an adhesive pad (CapSure™ Clean-up Pad, Arcturus), film from LCM MacroCap™ (Arcturus), heated film from LCM MacroCap™ (Arcturus) and employing a small gauge needle (for example, 28 gauge), to collect micro-cores of skin tissue.

The step of detecting the presence of mutations in the mtDNA can be selected from any technique as is known to those skilled in the art. For example, analyzing mtDNA can comprise sequencing the mtDNA, amplifying mtDNA by PCR, Southern, Northern, Western South-Western blot hybridizations, denaturing HPLC, hybridization to microarrays, biochips or gene chips, molecular marker analysis, biosensors, melting temperature profiling or a combination of any of the above.

In one aspect, the invention provides a method of detecting UV exposure in a mammal, the method comprising assaying a tissue sample from the mammal for the presence of an aberrant mitochondrial DNA described above. The present invention also provides for methods comprising assaying a tissue sample from the mammal by hybridizing the sample with at least one hybridization probe. The probe may be generated against a mutant mitochondrial DNA sequence of the invention as described herein.

In another aspect, the invention provides a method as above, wherein the assay comprises: a) conducting a hybridization reaction using at least one of the probes of the invention to allow the at least one probe to hybridize to a complementary aberrant mitochondrial DNA sequence; b) quantifying the amount of the at least one aberrant mitochondrial DNA sequence in the sample by quantifying the amount of the mitochondrial DNA hybridized to the at least one probe; and, c) comparing the amount of the mitochondrial DNA in the sample to at least one known reference value.

Also included in the present invention are methods for detecting, diagnosing or monitoring UV damage or exposure comprising diagnostic imaging assays as known in the art. The diagnostic assays of the invention can be readily adapted for high-throughput. High-throughput assays provide the advantage of processing many samples simultaneously and significantly decrease the time required to screen a large number of samples. The present invention, therefore, contemplates the use of the nucleotides of the present invention in high-throughput screening or assays to detect and/or quantitate target nucleotide sequences in a plurality of test samples.

### Fusion Transcripts

Another aspect of the invention is the identification of fusion transcripts and associated hybridization probes useful in methods for predicting, diagnosing and/or monitoring UV damage or exposure. As disclosed in the Applicant's co-pending PCT application, such molecules may be derived through the isolation of naturally-occurring transcripts or, alternatively, by the recombinant expression of mtDNAs isolated according to the methods of the invention. These mtDNAs typically comprise a spliced gene having the initiation codon from the first gene and the termination codon of the second gene. Accordingly, fusion transcripts derived therefrom include a junction point associated with the spliced genes.

### Detection of Fusion Transcripts

Naturally occurring fusion transcripts can be extracted from a biological sample and identified according to any suitable method known in the art, or may be conducted according to the methods described in the Applicant's co-pending PCT application. In one embodiment of the invention, stable polyadenylated fusion transcripts are identified using Oligo(dT) primers that target transcripts with poly-A tails, followed by RT-PCR using primer pairs designed against the target transcript.

The following exemplary fusion transcripts were detected using these methods:
SEQ ID NO: 22 (Transcript 2; 10744:14124)
SEQ ID NO: 38 (Transcript 20; 8469:13447; OrigMet)

Fusion transcripts can also be produced by recombinant techniques known in the art. Typically this involves transformation (including transfection, transduction, or infection) of a suitable host cell with an expression vector comprising an mtDNA sequence of interest.

### Detection of Unexpected Fusion Transcripts

Fusion transcripts of an unexpected nature have also been identified by the Applicant and proven useful in the methods of the present invention. Until now, each of the fusion transcripts identified occurred as a result of a deletion event in the mitochondrial genome resulting in the fusion of two genes to form a novel sequence then transcribed by the mitochondria. By contrast, the 3895bp deletion described in detail below, results in the fusion of the D-loop, which is located in a non-coding region of the mtDNA.

### Why detection of 3895bp deletion associated fusion transcript is unexpected

The majority of mitochondrial fusion transcripts are formed by the deletion of nucleotide content between two adjacent or non-adjacent genes (see *Genomic Mutations* section above and the Applicant's co-pending PCT application). After removal of the deleted DNA, the remainder of the mitochondrial genome is then recombined resulting in a new gene, or transcript. As previously discussed, this spliced gene is comprised of the initiation codon of the 5'-most original gene, varying contributions of genetic content from the original two genes, and the termination codon of the 3'-most original gene.

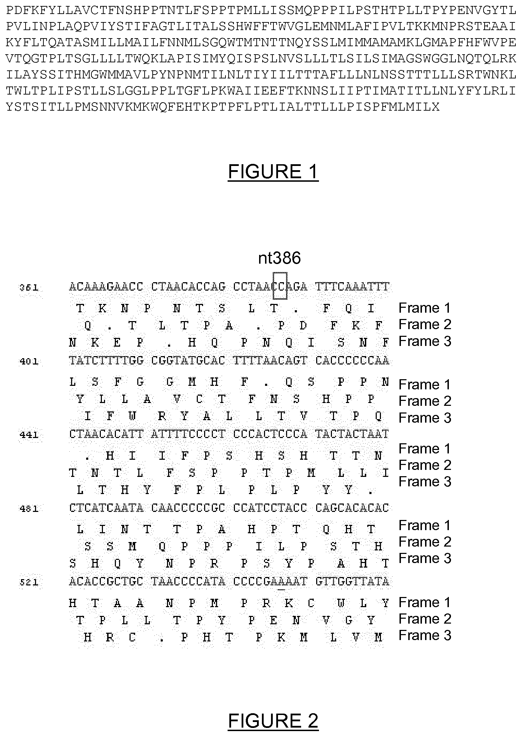

By contrast, the fusion transcript from the deletion of the genetic content between nucleotide positions 547-4443 (Transcript 32; SEQ ID NO: 39) is unexpected, since the 5' portion is located within the non-coding Displacement Loop (D-Loop) (16024-576) of the mitochondrial genome. Despite this fact, when positions 548-4442 are removed an intact reading frame in Frame 2 is observed from positions 386-547:4443-5511 (see Figure 1 & 2). Frames 1 and 3 have many termination codons, thus failing to produce a reading frame (Figure 2).

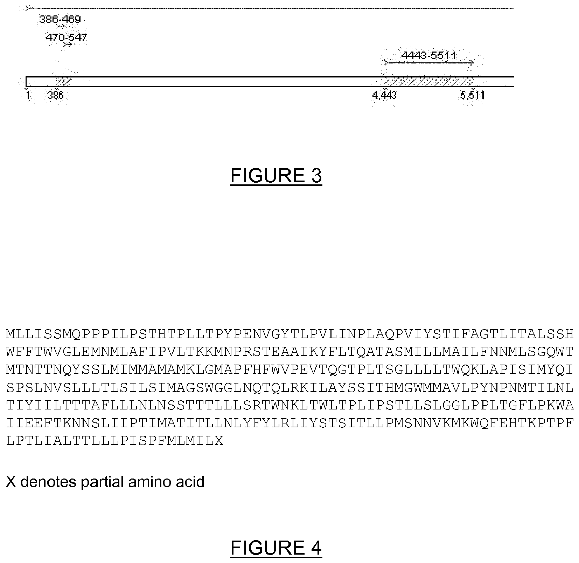

The 5' contents of the Frame 2 fusion transcript starts with proline at position 386 but only initiates at the first methionine located at position 470 located within the Hypervariable segment 3 (438-574). The 3' content of the fusion transcript begins at position 4443 within tRNA methionine (4402-4469) and terminates at position 5511 of NADH dehydrogenase subunit 2 (4470-5511) (Figure 3). Figure 4 displays the resulting fusion transcript initiating at position 470 formed by the deletion of positions 548-4442 of the mitochondrial genome.

Accordingly, the present invention encompasses fusions transcripts of varied content, *i.e.* those comprising spliced sequences of two coding regions and those comprising spliced coding and non-coding regions.

### Fusion Transcripts Associated with UVR

Once mitochondrial fusion transcripts have been identified, the sequences are then tested for their association with UVR, as described in the examples below. Such testing may be carried out *in vivo,* or in cultured skin equivalents that are grown and dosed with varying levels of UVR (see Example 6). Expression of these sequences may also be monitored in respect of one or more house keeper transcripts including, but not limited to, Human PPIB, Human PGK-1, Human ACTB (beta actin),Human GUSB (Beta Glucuronidase), Human B2M (Beta-2-microglobulin), Human PPIA (peptidylprolyl isomerase A (cyclophilin A)), Human GAPD (glyeraldehyde-3-phosphate dehydrogenase), Human HPRT1 (hypoxanthine phosporibosyltransferase 1), Human RPLPO = LRP (ribosmal protein, large, PO), Human HMBS = PBGD (hydroxymethlbilane synthase), Human TBP (TATA box binding protein), Human TRFC (transferrin receptor (p90, CD71), and Human ABCC13 (Chromosome 13 - psuedogene).

In one embodiment of the invention, the following mitochondrial fusion transcripts have been determined useful for predicting, diagnosing or monitoring UV damage, and for testing and screening skin care products effective in preventing or ameliorating UV damage:
SEQ ID NO: 22 (Transcript 2; 10744:14124)
SEQ ID NO: 23 (Transcript 3; 7974:15496)
SEQ ID NO: 31 (Transcript 11; 7775:13532)
SEQ ID NO: 32 (Transcript 12; 8213:13991)
SEQ ID NO: 38 (Transcript 20; 8469:13447; OrigMet)
SEQ ID NO: 39 (Transcript 32; 547:4443)

In addition to the above sequences, the present invention provides the use of variants or fragments of these sequences for predicting, diagnosing and/or monitoring UV exposure. Variants or fragments of the fusion transcripts identified herein adhere to the size limitations and percent identities described above with respect to the genomic variants and fragments, or as determined suitable by a skilled technician.

In addition, putative protein sequences corresponding to transcripts 2, 3, 11, 12, 20 and 32 of the invention are listed below. These sequences, which encode hypothetical fusion proteins, are provided as a further embodiment of the present invention and may be considered useful in the methods of the present invention.
SEQ ID NO: 40 (Transcript 2)
SEQ ID NO: 41 (Transcript 3)
SEQ ID NO: 42 (Transcript 11)
SEQ ID NO: 43 (Transcript 12)
SEQ ID NO: 44 (Transcripts 20)
SEQ ID NO: 45 (Transcript 32)

### Primers and Probes

Once a fusion transcript has been characterized, primers or probes can be developed to target the transcript in a biological sample. Such primers and probes may be prepared using any known method (as described above) or as set out in the examples provided in the Applicant's co-pending PCT application. A probe may, for example, be generated for the fusion transcript, and detection technologies, such as QuantiGene 2.0™ by Panomics™, used to detect the presence of the transcript in a sample. Primers and probes may be generated directly against exemplary fusion transcripts of the invention, or to a fragment or variant thereof. For instance, the sequences set forth in SEQ ID NOs: 22, 23, 31, 32, 38 and 39 can be used to design probes that will detect a nucleic acid sequence comprising a fusion sequence of interest.

As would be understood by those skilled in the art, probes designed to hybridize to the fusion transcripts of the invention contain a sequence complementary to at least a portion of the transcript and preferably expressing the junction point of the spliced genes. This portion includes at least one of the nucleotides complementary to the expressed junction point, and may further comprise one or more complementary nucleotides adjacent thereto. In this regard, the present invention encompasses any suitable targeting mechanism that will select a fusion transcript that uses the nucleotides involved and adjacent to the junction point of the spliced genes.

Various types of probes and methods of labelling known in the art are contemplated for the preparation of transcript probes. Such types and methods have been described above with respect to the detection of genomic sequences. The transcript probes of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A probe of "at least 20 nt in length," for example, is intended to include 20 or more contiguous bases that are complementary to an mtDNA sequence of the invention. Of course, larger probes (e.g., 50, 150, 500, 600, 2000 nucleotides) may be preferable.

The probes of the invention will also hybridize to the fusion transcripts in biological samples, thereby enabling the methods of the invention. Accordingly, in one aspect of the invention, there is provided a hybridization probe for use in detecting, diagnosing and/or monitoring UV damage or exposure, wherein the probe is complementary to at least a portion of a fusion transcript of the invention. In another aspect, there is provided probes and use thereof (or a method of using) such probes for testing and screening skin care products effective in preventing or ameliorating UV damage or exposure.

### Assays

In accordance with the present invention, novel fusion transcripts have been demonstrated to increase in abundance following exposure to irradiation with solar simulated light (see, for instance, Example 6). Detection of these novel fusion transcripts both *in vitro* and *in vivo* allows for the quantification of exposure to UV irradiation (both UVB and UVA). The present invention, therefore, encompasses methods for detecting, diagnosing or monitoring UV damage or exposure, comprising obtaining one or more biological samples, extracting mitochondrial RNA from the samples, and assaying the samples for fusion transcripts by: quantifying the amount of one or more fusion transcripts in the sample and comparing the quantity detected with a reference value.

As would be understood by those of skill in the art, the reference value is based on whether the method seeks to detect, diagnosis or monitor UV damage or exposure. Accordingly, the reference value may relate to transcript data collected from one or more known UV exposed biological samples, from one or more known non-UV exposed biological samples, and/or from one or more biological samples taken over time. The sample may be derived from rarely sun exposed, occasionally sun exposed, usually sun exposed skin or blood and collected by a variety of methods as indicated above.

In one aspect, the invention provides a method of detecting UV exposure in a mammal, the method comprising assaying a tissue sample from the mammal for the presence of a fusion transcript described above. The present invention also provides for methods comprising assaying a tissue sample from the mammal by hybridizing the sample with at least one hybridization probe. The probe may be generated against a fusion transcript of the invention as described herein.

In another aspect, the invention provides a method as above, wherein the assay comprises: a) conducting a hybridization reaction using at least one of the probes to allow the at least one probe to hybridize to a complementary fusion transcript sequence; b) quantifying the amount of the at least one fusion transcript sequence in the sample by quantifying the amount of the transcript hybridized to the at least one probe; and, c) comparing the amount of the transcript in the sample to at least one known reference value.

As discussed above, the diagnostic assays of the invention may also comprise diagnostic methods and screening tools as described herein and can be readily adapted for high-throughput. The present invention, therefore, contemplates the use of the fusion transcripts and associated probes of the present invention in high-throughput screening or assays to detect and/or quantitate target nucleotide sequences in a plurality of test samples.

### Testing and Screening of Skin Care Products

### New Formulations

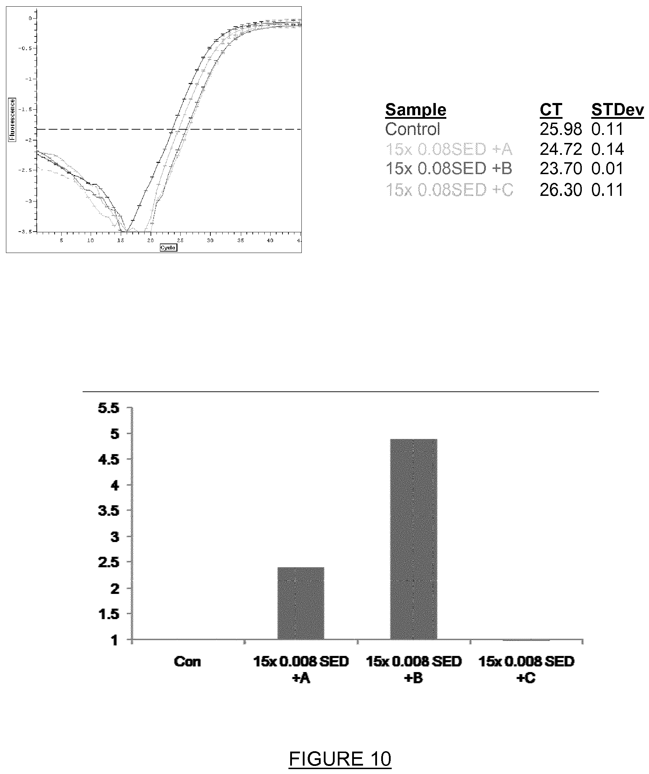

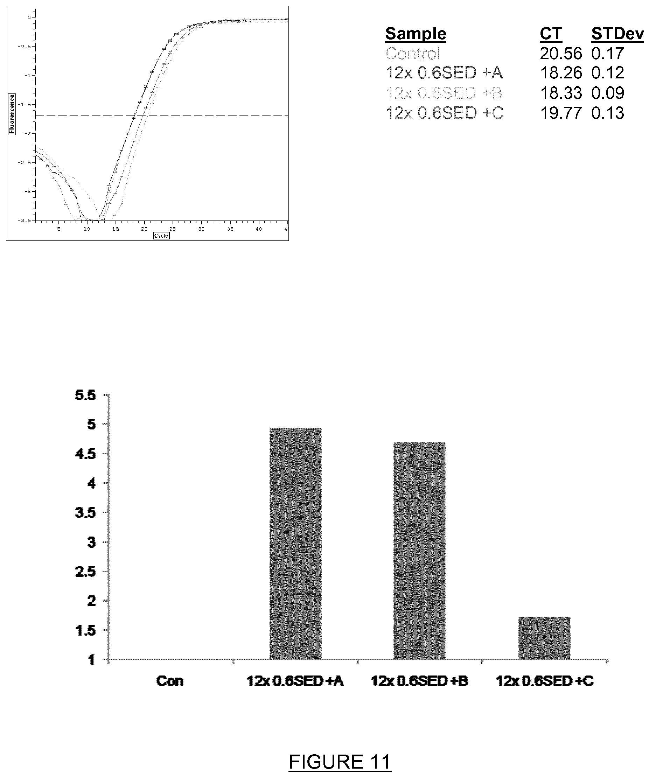

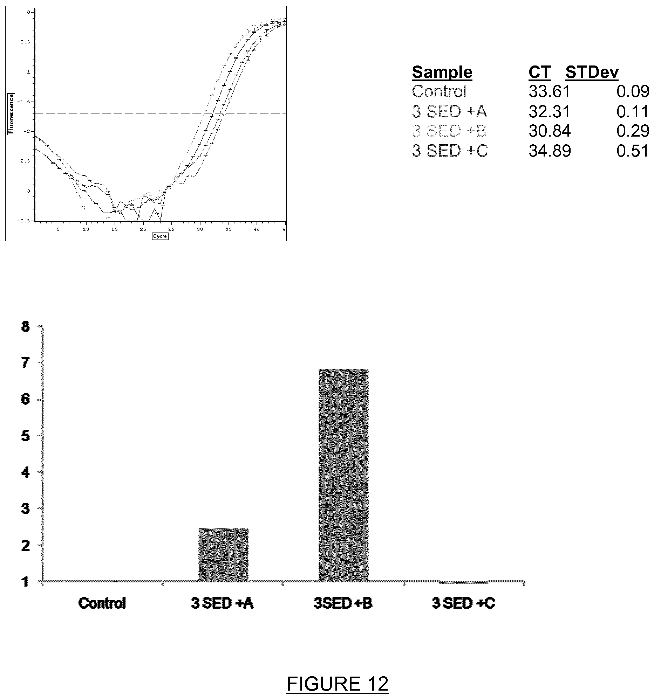

Modulation of changes in the abundance of novel UV associated fusion transcripts and related mtDNA molecules, through the application of specific actives or formulations such as sunscreens, anti-oxidant or anti-ageing products, may be used to assess the efficacy of products in protecting the skin from damage by UV irradiation. Such assessment may take the form of *in vitro* testing of new formulations using Skin Equivalent Models (see Figures 10 and 11) or through *in vivo* testing of products using biological samples (see Figure 12).

The invention therefore contemplates methods of preparing and testing the efficacy of a skin care product to prevent, minimize, ameliorate or protect against UV exposure or damage by preparing a product having the desired characteristics (e.g. UVA filter and/or UVB filter), applying the skin care product to a patient's skin or to a skin equivalent model; exposing the skin or skin equivalent model to UVR; detecting the presence of at least one mitochondrial deletion molecule and/or fusion transcript in a sample taken from the exposed patient's skin or the exposed skin equivalent; and comparing the presence of the deletion or transcript to a reference value, such as a control gene and/or transcript. Control patients or skin equivalents models, which have not received the skin product, may also be used as a reference value.

The skilled person would understand that the arrest, prevention, decrease, or improvement in one or more the symptoms, signs, or features of UV damage, both temporary and long-term, indicates the ability of the new product (formulation) to prevent, minimize, ameliorate or protect against UV exposure or damage. In one embodiment of the invention, product analysis is performed as shown in the examples provided below.

In preferred embodiments of the invention, the methods for testing the new products comprise exposing a subject's skin or a skin equivalent model to at least one sub-lethal dose of ultraviolet radiation (UVR) prior to the step of detecting the presence of the mtDNA deletion or fusion transcript. Further, the skin or skin equivalent may be exposed to a series of repetitive sub-lethal doses of UVR, such as daily doses of UVR, prior to testing. Generally, the UVR is from a solar-simulated UVR source, wherein the UVR comprises UVA, UVB, or UVA/UVB, however, sun exposure is also herein contemplated. Also contemplated are control sequences including, but not limited to, the housekeeping genes and transcripts discussed above. As for the mtDNA deletion and fusion transcript markers, these can be inserted at the end of the described methods either singularly or in tandem.

### Skin Samples

The patient's skin sample may be collected from the dermal or epidermal layer of the skin and may be derived by way of punch biopsy, surgical excision, and non-invasive or minimally invasive skin sampling methods such as a wet swabbing, tapelift, cotton tip swabbing, scraping of skin using a sterile surgical blade, scraping of skin using a wooden scraper, sticky surface of an adhesive pad (CapSure™ Clean-up Pad, Arcturus), film from LCM MacroCap™ (Arcturus), heated film from LCM MacroCap™ (Arcturus) and employing a small gauge needle (for example, 28 gauge), to collect micro-cores of skin tissue. The sample can be used either directly as obtained from the source or following a pre-treatment to modify the character of the sample. Thus, the skin sample can be pre-treated prior to use, for example, with preservatives, reagents, and the like.

One skilled in the art will appreciate that more than one sampling technique may be employed at a single time. Furthermore, where a course of collections are required, for example, for the monitoring of a product over time, the same or different techniques may be used alone or together throughout the test period. In this regard, skin collections may be taken once only, or at regular intervals such as daily, weekly or monthly.

### Skin Equivalent Models

The Applicant has also developed a novel system for testing skin care products *in vitro* using Skin Equivalent Models. These models are produced and grown, for instance, as described in Example 2, following which they may be treated with a skin care product and dosed by varying increments of UVR. The product to be tested is simply applied to the surface of the skin equivalent, for example, at a density of 2mg/cm², and spread evenly across the surface of the cells. The skin equivalents are then placed under the solar simulator and exposed to one or more doses of UV light. Dosing is dependent on the individual experimental setup and product to be tested. However, several examples of typical dosing regimes are provided in Tables 1 and 2 below.

### Product Screening and Individualized Skin Care

Through the collection of data on the ability of skin care products to protect against the generation of fusion transcripts and related mtDNA deletions, more effective products with regard to sun protection and anti-ageing can be developed. As well, until more recently, sunscreens and sunblocks regularly applied by individuals during sun exposure generally protected against the mutagenizing effects of UVB, but failed to contain agents directed at the harmful effects of UVA radiation. Thus, targeted screening of skin care products will aid in identifying those capable of protecting against both UVA and UVB radiation.

These factors among others necessitate the availability of a tool to determine both the efficacy of new products entering the market and, through a course of studies, monitor the success and appropriateness of current measures to prevent UVR damage to the skin. Measuring the level of mitochondrial DNA deletions in the skin of a patient or skin equivalent model by collecting skin samples following product application and UVR dosing at regular intervals can assist with determining the efficacy of such products for the prevention of DNA and skin damage, including associated photoaging and skin cancer.

The methods of the present invention may also be used for widespread skin screening for both medical and cosmeceutical purposes. For example, the ability to assess the level of DNA damage in a subject's skin due to UV radiation at any time point and from any external anatomical location provides the foundation for a unique and informative screening test to assess the safety and efficacy of existing and new skin care products and skin care regimes for a given subject. Furthermore, by identifying the specific genetic changes associated with UV exposure, it may be readily determined whether and to what extent a particular skin care product or regime should be applied.

For skin care products already on the market, the methods of the present invention also assist in screening agents to gauge their ability to prevent, minimize, ameliorate or protect against UV exposure or damage. This allows a practitioner or consumer to assess which brands are best suited for their particular skin care requirements. Products to be screened by the methods of the invention include but are not limited to sunscreens and anti-aging serums and creams and maybe assessed in pairs or in batches of 3 or more products. Screening methods may be carried out as described above for testing new formulations or as detailed in the examples below.

### Kits

The present invention provides diagnostic kits for detecting or monitoring the UV exposure of a subject. Such kits may include one or more sampling means, in combination with one or more primers or probes according to the present invention. Such kits may also include instructions for using the contents thereof.

The kits can optionally include reagents required to conduct a diagnostic assay, such as buffers, salts, detection reagents, and the like. Other components, such as solutions for the isolation and/or treatment of a biological sample, may also be included in the kit. One or more of the components of the kit may be lyophilised and the kit may further comprise reagents suitable for the reconstitution of the lyophilised components.

Where appropriate, the kit may also contain reaction vessels, mixing vessels and other components that facilitate the preparation of the test sample. The kit may also optionally include instructions for use, which may be provided in paper form or in computer-readable form, such as a disc, CD, DVD or the like.

In one embodiment of the invention there is provided a kit for diagnosing UV exposure comprising sampling means and a probe or primer of the invention.

To gain a better understanding of the invention described herein, the following examples are set forth. It will be understood that these examples are intended to describe illustrative embodiments of the invention and are not intended to limit the scope of the invention in any way.

### Examples

### Example 1: Growth of HpEKp Cells

### Materials

1. HpEKp Cells <15 passage (CellnTec)
2. T75 Tissue Culture Flasks (IWAKI)
3. TrypLE™ Select (12563-011, Invitrogen)
4. 10ml Sterile Stripettes (Star Labs)
5. Automated Pipetter
6. Inverted TC microscope
7. Sterile 15ml Falcon Tube (Falcon)
8. Class 2 Tissue Culture Cabinet [37°C, 5%CO²] (Binder)
9. Water Bath (37°C)
10. Sterile Phosphate buffered Saline
11. Complete PCM Medium [CnT-57] (CellnTec)

### Protocol

1. Remove CnT-57 medium from flasks of HpEKp cells (90% confluence)
2. Wash cells with sterile PBS (2ml), aspirate off.
3. Add 1.5ml TrypLE™ Select per flask, to cover cells.
4. Place back in incubator for 2-3 minutes, until cells become detatched (check with microscope).
5. Resuspend Cells in 5ml of CnT-57 Medium to resuspend cells, vigorously pipette 2/3 times.
6. Spin the cells at 160xg for 5 min.
7. Remove medium and resuspend in 5ml of fresh CnT-57 Medium, vigorously pipette 2/3 times.
8. Remove 20ul cell suspension for cell counting.
9. Dilute cells and seed to a concentration of 4x10³ cells/cm²
10. Place Flasks in a humidified incubator at 37°C and 5% CO2
11. Maintain cells by feeding with 15ml CnT-57 medium every 2/3 days.
12. Grow until 90% confluent then passage again.

### Example 2: Epidermal Skin Equivalent Production

### Materials

1. HpEKp Cells <15 passage (CellnTec) grown to 90% confluence (see Example 1)
2. T75 Tissue Culture Flasks (IWAKI)
3. TrypLE™ Select (12563-011, Invitrogen)
4. 10ml Sterile Stripettes (Star Labs)
5. Automated Pipetter
6. Inverted TC microscope
7. Sterile 15ml Falcon Tube (Falcon)
8. 6 Well Culture Plate (Millipore)
9. Millicell PCF 0.4 µm Inserts (Millipore)
10. RapiDiff II stain Pack (BioStain)
11. 24 Well Culture Plates (Millipore)
12. Class 2 Tissue Culture Cabinet [37°C, 5%CO2] (Binder)
13. Water Bath (37°C)
14. Sterile Phosphate buffered Saline
15. Sterile Forceps
16. Complete PCM Medium [CnT-57] (CellnTec)
17. Complete 3D-Prime Medium [CnT-02-3DP] (CellnTec)

### Protocol

1. Place four Millicell PCF 0.4 µm 12mm inserts (Millipore Cat#: PIHP01250) into each well of a 6 well culture plate, plating out the number required for the experiment. Allow for two spare inserts (to monitor confluency - step 15).
2. Remove CnT-57 medium from flasks of HpEKp cells.
3. Wash cells with sterile PBS (2ml), aspirate off.
4. Add 1.5ml TrypLE™ Select per flask, to cover cells.
5. Place back in incubator for 2-3 minutes, until cells become detached (check with microscope).
6. Re-suspend Cells in 5ml of CnT-57 Medium to re-suspend cells, vigorously pipette 2/3 times.
7. Spin the cells at 160xg for 5 min.
8. Remove medium and re-suspend in 5ml of fresh CnT-57 Medium, vigorously pipette 2/3 times.
9. Remove 20ul cell suspension for cell counting.
10. Dilute cells to a concentration of 5x 10₅ cells per ml.
11. Add 2x105 cells in 400µl CnT-57 (or CnT-07) per insert.
12. Add the appropriate amount (∼2ml) of CnT-57 outside the inserts (into the plate well), so that medium levels inside and outside the insert are equal and submerge the cells; make sure that no air bubbles are trapped underneath the membrane.
13. Place the inserts in a humidified incubator at 37°C and 5% CO2
14. Allow the cells to grow for 2-3 days.
15. Stain Single spare insert with RAPI-DIFF II stain.
16. If the monolayer is confluent, proceed with step 17, otherwise change medium in the remaining inserts, cultivate for another day and perform then another staining with the second spare insert.
17. Replace the culture medium with 3D medium (CnT-02-3DP) inside and outside the insert (same amounts as when seeding, Step 12).
18. Place the inserts in the incubator overnight (15-16h) to allow cells to form intercellular adhesion structures.
19. Initiate 3D cultures by aspirating all the medium from inside the insert and place into individual wells of a 24 well plate. Add (CnT-02-3DP) outside medium (250µl)
20. If a time course study is performed, inserts can be left in the same plate with medium changes every 2-3 days. Grow cells for 14 days air dry prior to beginning dose treatments.

### Example 3: Epidermal Skin Equivalent Dosing - UVA Irradiation

### Materials

1. Oriel 1000W UV Solar Simulator (*Newport Corp*.)
2. Atmospheric Attenuation Filter (Newport Corp. - 81017)
3. Vis IR Filter (Newport corp - 87066)
4. PETG Filter (RVI Medical Physics)
5. International Light UVA Phototherapy Radiometer (Able Instruments - IL1402)
6. Epidermail Skin equivalent (See Example 2)
7. Complete 3D-Prime Medium [CnT-02-3DP] (CellnTec)
8. 24 Well Culture plate (Millipore)
9. Sterile Phosphate buffered Saline
10. Sterile Forceps and Scalpel

### UV Spectrum Used (Solar Simulated)

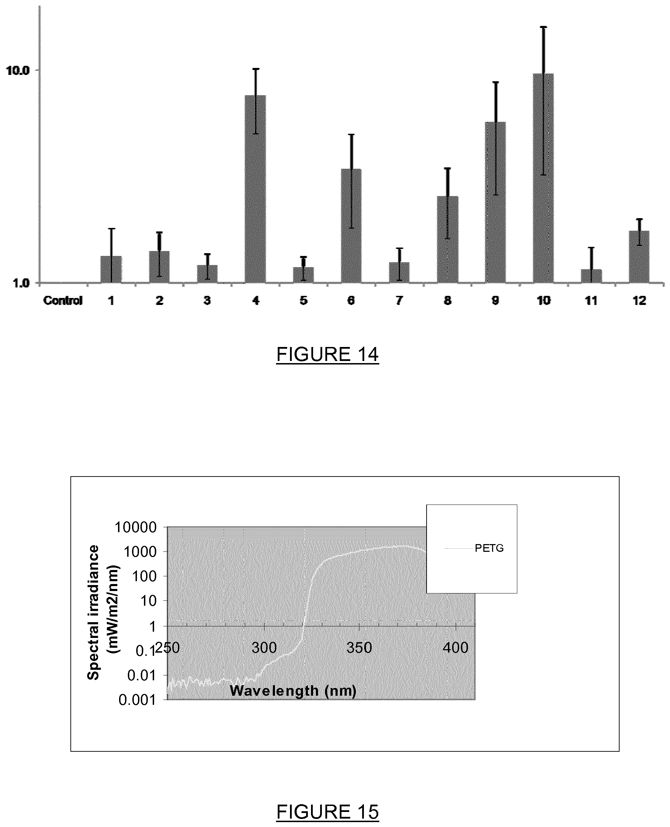

Atmospheric Attenuation filter + Vis IR filter + PETG Filter: As shown in Figure 15.

### Procedure

1. Skin Equivalents produced and grown as set out in Example 2.
2. After 14 days of air dry growth the SEs are ready for dosing/treatment.
3. Skin equivalents are removed from the growth medium and placed in new 24 well plate, containing 200µl of sterile PBS.
4. If required the product to be tested is applied to the surface of the skin equivalent, at a density of 2mg/cm² (or at any specified dose). The material is applied with a bent sterile 200µl pipette tip, and spread evenly across the surface.
5. Once the skin equivalent is prepared, they are placed back in the incubator for 20 minutes prior to exposure to UV light.
6. The solar simulator is turned on and allowed to warm up for 10 minutes prior to treatment. The UV output is measured with the radiometer and the intensity is used to calculate the required time of exposure.
7. The skin equivalents are placed under the solar simulator and exposed to X SED of UV light (as calculated in 6 dependant on experimental requirements).
8. Following exposure the skin equivalents are replaced into the 3D medium and back into the incubator.
9. Dosing is dependent on the individual experimental setup. Some examples are shown in table 1.
10. Following dosing SEs are removed from the 3D medium and stored at -80°C prior to extraction.

**Table 1**

| **Experimental Setup** |
|---|
| Dose Response 0.04 SED, multiple doses |
| Dose Response 0.08 SED, multiple doses |
| SPF 15 Products 0.08 SED, multiple doses |
| Anti-Aging products 0.08 SED, multiple doses |

### Example 4: Epidermal Skin Equivalent Dosing - Solar Simulated light

### Materials

1. Oriel 1000W UV Solar Simulator (*Newport Corp*.)
2. Atmospheric Attenuation Filter (*Newport Corp. - 81017*)
3. Vis IR Filter (*Newport corp - 87066*)
4. International Light UVA Phototherapy Radiometer *(Able Instruments -* IL1402)
5. Epidermail Skin equivalent (*See Example 2*)
6. Complete 3D-Prime Medium [CnT-02-3DP] *(CellnTec)*
7. 24 Well Culture plate *(Millipore)*
8. Sterile Phosphate buffered Saline
9. Sterile Forceps and Scalpel

### UV Spectrum Used (Solar Simulated)

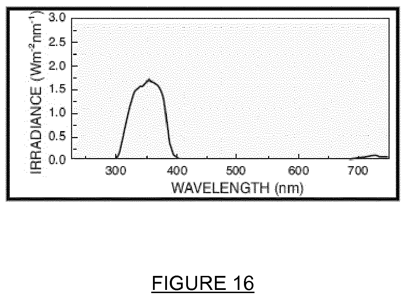

Atmospheric Attenuation filter + Vis IR filter: as shown in Figure 16.

### Procedure

1. Skin Equivalents produced and grown as set out in Example 2.
2. After 14 days of air dry growth the SEs are ready for dosing/treatment.
3. Skin equivalents are removed from the growth medium and placed in new 24 well plate, containing 200µl of sterile PBS.
4. If required the product to be tested is applied to the surface of the skin equivalent, at a density of 2mg/cm² (or at any specified dose). The material is applied with a bent sterile 200µl pipette tip, and spread evenly across the surface.
5. Once the skin equivalent is prepared, they are placed back in the incubator for 20 minutes prior to exposure to UV light.
6. The solar simulator is turned on and allowed to warm up for 10 minutes prior to treatment. The UV output is measured with the radiometer and the intensity is used to calculate the required time of exposure.
7. The skin equivalents are placed under the solar simulator and exposed to X SED of UV light (as calculated in 6 dependant on experimental requirements).
8. Following exposure the skin equivalents are replaced into the 3D medium and back into the incubator.
9. Dosing is dependent on the individual experimental setup. Some examples are shown in table 1.
10. Following dosing SEs are removed from the 3D medium and stored at -80°C prior to extraction.

**Table 2**

| **Experimental Setup** |
|---|
| Dose Response 0.5 SED, multiple doses |
| Dose Response 1 SED, multiple doses |
| SPF 15 Products 1 SED, multiple doses |
| Anti-Aging products 0.5 SED, multiple doses |

### Example 5: In vivo UVR Dose Response

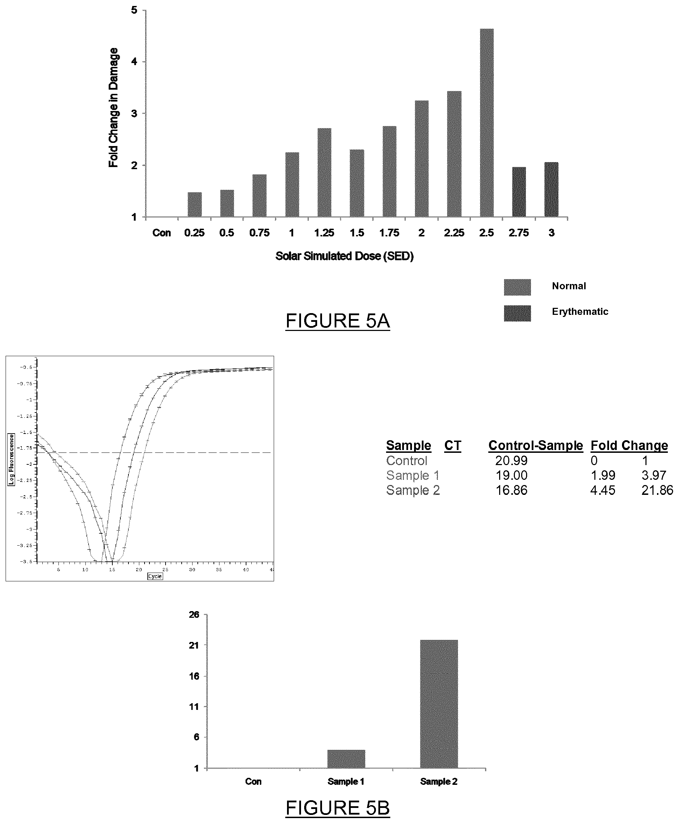

*In vivo* testing was carried out on individual(s) by dosing with various levels of UVR followed by swabbing to collect skin samples. Referring to Figure 5, SED refers to Standard Erythemal Dose, which is the amount of UVR required to cause erythema (or reddening of the skin). Figure 5A shows the dose response results for mtDNA deletion analysis performed by quantative realtime PCR on skin swab samples taken following UVR doses of up to 3.0 SED. This experiment demonstrates that UVR induced mtDNA damage increases with increasing doses of UVR, up to the point where erythema begins to occur. At this point the mtDNA damage in the sample falls, possibly due to increased levels of apoptosis in the erythemic tissue. In terms of long term damage to skin this shows that it is sub erythemal doses which create the long term mtDNA damage observed in skin. The fold change in damage is calculated as set out in Figure 5B.

### Example 6: Identification of Fusion Transcripts Associated with UV Exposure

Cultured skin equivalents were grown and dosed with varying levels of UVR using a solar simulator as described in the examples above.

Samples were processed according to the manufacturer's protocol QuantiGene Sample Processing Kit: Cultured Cells (Panomics QS0100) and the QuantiGene 2.0 Reagent System (Panomics QS0008), and specifically 300ul of dilute lysis mixture was added to each skin equivalent which were then incubated at 50 degrees Celsius for 1.5 hours or until the cells appeared to have completely lysed off of the membrane. Each was then diluted 1:10 and the remainder of the protocol carried out, targeting fusion transcripts 2, 3, 11, 12, 20 and 32 with ACTB Beta-Actin as the Housekeeper. Each sample was assayed in triplicate for all transcripts and a no-template background was established for each sample/transcript pair in triplicate as well.

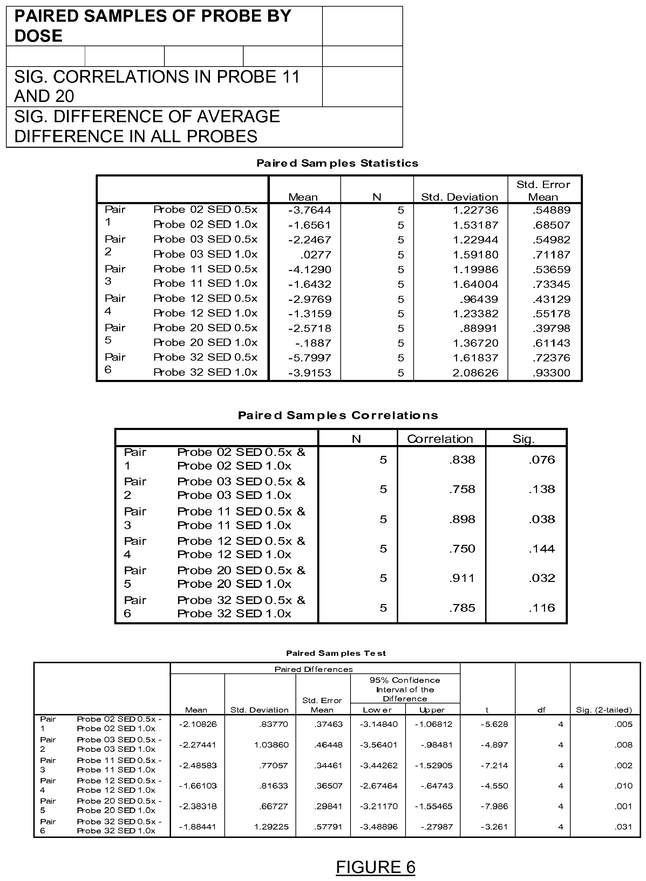

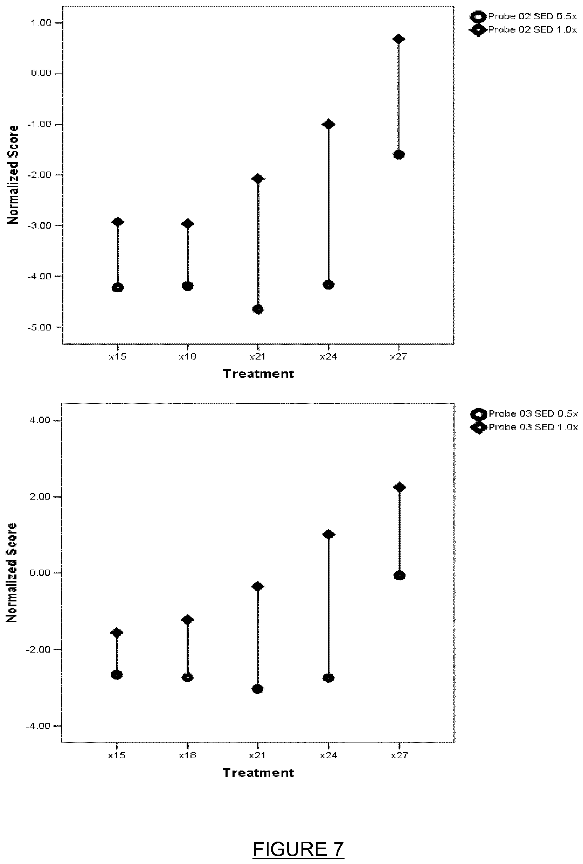

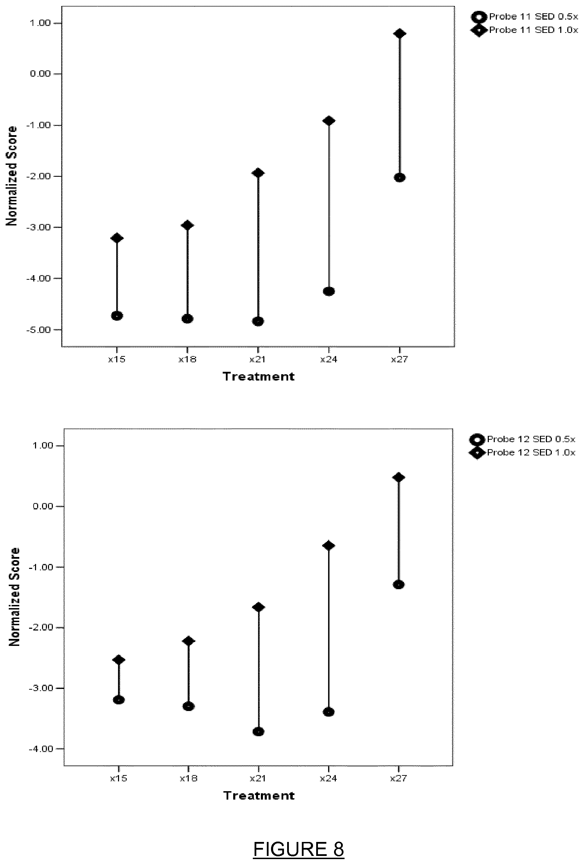

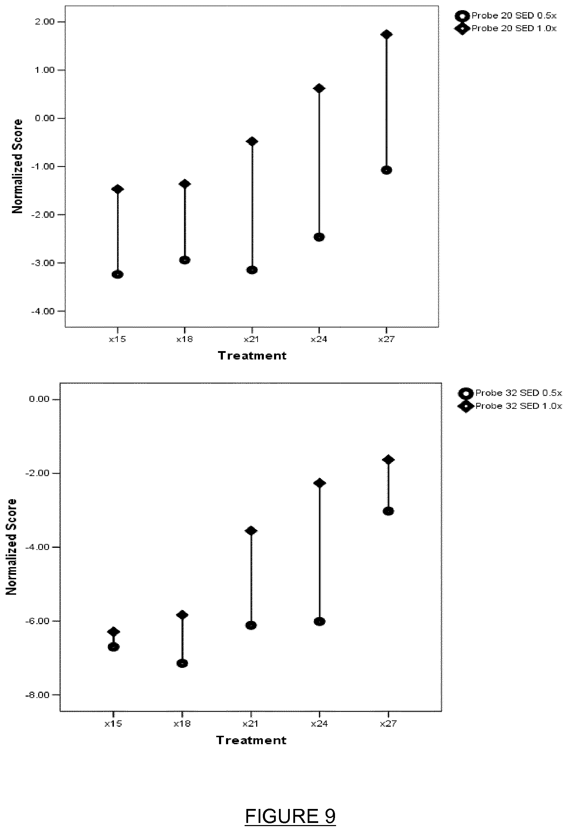

Referring to Figures 6-9, 0.5 SED and 1.0 SED were used for the purposes of this experiment at a various number of repeated doses (0X, 15X, 18X, 21X, 24X, 27X). For clarity, those skin equivalents exposed to 1.0 SED 27X received the greatest level of UVR.

These results demonstrate that each of the fusion transcripts tested increased in quantity both with increasing repeat doses as well as between the 0.5 and 1.0 SED indicating an association with UVR exposure.

### Example 7: Product Formulation and Testing

Three blinded formulations of varying levels of UVA filter were tested at 2mg/cm2. Samples of each formulation were tested on 14 day old Skin Equivalents with both UVA and Solar Simulated light sources. As well, the samples were tested *in vivo.* Deletion analysis was performed by sybr green quantitative realtime PCR.

The experimental formulations were prepared in a cosmetic lotion emulsion comprised of standard cosmetic ingredients. Each product contains a constant level of UVB filter but levels of UVA filter were varied as follows:

**Table 3: Content of new formulations**

| **Sample No.** | **UVB Filter** | **UVA Filter** | **Star Rating** |
|---|---|---|---|
| 1 (**B** in figures) | 3.8% | 0% | 1 Star |
| 2 (**A** in figures) | 3.8% | 1.75% | 3 Star |
| 3 (**C** in figures) | 3.8% | 4.5% | 5 Star |

Figure 10 shows the results of PCR analysis on *in vitro* samples exposed to UVA (Figure 10) and solar simulated light (Figure 11) following application of the formulations (A, B or C). In Figure 12, the results following *in vivo* testing of the formulations are provided. These data show the multifactoral nature of UVR damage from solar simulated light, when isolated with only UVA exposure, damage levels follow the percentage of UVA protection afforded by the sunscreen. However when the exposure combines both UVA and UVB UVR in solar simulated light the damage ratios between samples changes. With respect to samples A and B, rather than A being higher than expected, B may be lower than expected as the lack of UVA filter combined with the UVB in the solar simulated light has taken the damage past the erythemic threshold (section 00121), meaning less damage is recorded in the sample.

These results also demonstrate that there exists an inverse correlation between the level of damage as indicated by the level of deletion and the amount of UVA filter in the compound. For clarity, less UVA filter, as in compound B, results in higher levels of the deletion.

### Example 8: Sunscreen and Anti-aging Brand Screening

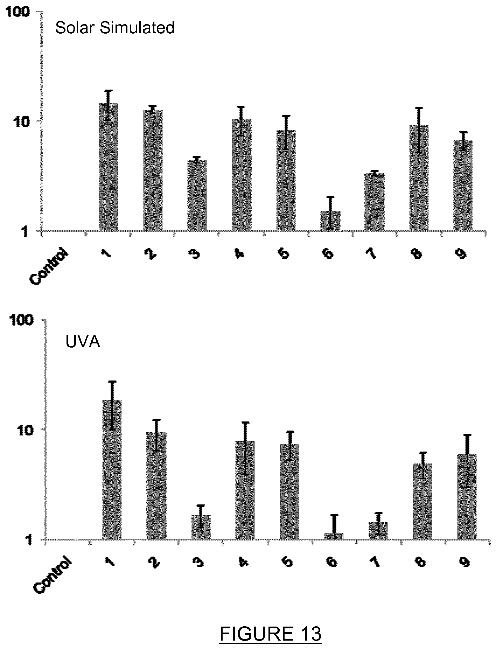

Samples of various sunscreen and anti-aging products were applied prior to repetitive exposure to UVA light at 2mg/cm2. The panel of sunscreen agents included spf 15 products from top UK/North American brands. Anti-aging products included a range of night crèmes/serums from a number of top brands (Figures 13). The results show that despite all products having the same SPF rating (15), the level of protection afforded by the products varied considerably, following dosing with both UVA and solar simulated UVR. This suggests that SPF alone is insufficient to assess the effectiveness of UVR protection afforded by sunscreens, as other factors other than UVB exposure (measured by SPF rating) are important in mtDNA damage, such as UVA protection and anti-oxidant activity. Anti-aging products included a range of night crèmes/serums from a number of top brands (Figure 14). These data show that there is great variation in the protection afforded by anti-aging type products as far as mtDNA damage is concerned. Many of these products contain anti-oxidant compounds as their main actives, and this shows that measurement of mtDNA damage can distinguish between those formulations and indeed individual actives which are effective and those which exhibit little to no protection.

Although the invention has been described with reference to certain specific embodiments, various modifications thereof will be apparent to those skilled in the art without departing from the purpose and scope of the invention as outlined in the claims appended hereto. Any examples provided herein are included solely for the purpose of illustrating the invention and are not intended to limit the invention in any way. Any drawings provided herein are solely for the purpose of illustrating various aspects of the invention and are not intended to be drawn to scale or to limit the invention in any way. The disclosures of all prior art recited herein are incorporated herein by reference in their entirety.

### Bibliography

The following references, amongst others, were cited in the foregoing description. The entire contents of these references are incorporated herein by way of reference thereto.

| **Author** | **journal** | **Title** | **Volume** | **Date** |
|---|---|---|---|---|
| Anderson et al | Nature | Sequence and Organization of the Human Mitochondrial Genome | 290(5806):457-65 | 1981 |
| Andrews et al | Nat Genet | Reanalysis and revision of the Cambridge reference sequence for human mitochondrial DNA. | 23(2):147 | 1999 |
| Modica-Napolitano et al | Expert Rev Mol Med | Mitochondria as targets for detection and treatment of cancer | 4:1-19 | 2002 |
| Sherratt et al | Clin Sci (Lond) | Mitochondrial DNA defects: a widening clinical spectrum of disorders. | 92(3):225-35 | 1997 |
| Croteau et al | Mutat Res | Mitochondrial DNA repair pathways. | 434(3):137-48 | 1999 |
| Dai et al | Acta Otolaryngol | Correlation of cochlear blood supply with mitochondrial DNA common deletion in presbyacusis. | 24(2):130-6 | 2004 |
| Ro et al | Muscle Nerve | Deleted 4977-bp mitochondrial DNA mutation is associated with sporadic amyotrophic lateral sclerosis: a hospital-based case-control study. | 28(6):737-43 | 2003 |
| Barron et al | Invest Ophthalmol Vis Sci | Mitochondrial abnormalities in ageing macular photoreceptors. | 42(12):3016-22 | 2001 |
| Lewis et al | J Pathol | Detection of damage to the mitochondrial genome in the oncocytic cells of Warthin's tumour. | 191(3):274-81 | 2000 |
| Muller-Hocker et al | Mod Pathol | The common 4977 base pair deletion of mitochondrial DNA preferentially accumulates in the cardiac conduction system of patients with Kearns-Sayre syndrome. | 11(3):295-301. | 1998 |
| Porteous et al | Eur J Biochem | Bioenergetic consequences of accumulating the common 4977-bp mitochondrial DNA deletion. | 257(1): 192-201 | 1998 |
| Birch-Machin MA | Online Conference Report (Sunburnt DNA) | International Congress of Biochemistry and Molecular Biology, New Scientist | | 2000(a) |
| Lee HC et al. | Federation of European Biochemical Societies | Aging-and smoking-associated alteration in the relative content of mitochondrial DNA in human lung | 441:292-296 | 1998 |
| Polyak Y. et al. | Nature Genetics | Somatic mutations of the mitochondrial genome in human colorectal tumours | 20 (3):291-293 | 1998 |
| Rees JL | The Genetic Basis of Human Cancer | Skin Cancer | pp. 527-536 | 1998 |
| Weinstock MA | Epidemiology | Epidemiology of Ultraviolet Radiation | pp. 121-128 | 1998 |

## Claims

1. A method of testing or screening the ability of a skin care product in preventing, minimizing, ameliorating or protecting against UV exposure, UV damage, skin aging or photo-aging, the method comprising the steps of;
(a) applying the skin care product to a test sample, the test sample comprising skin or a skin equivalent;
(b) exposing the test sample to ultraviolet radiation (UVR);
(c) detecting in the test sample the presence of:
(i) a mitochondrial fusion transcript associated with UV exposure, wherein the mitochondrial fusion transcript comprises the nucleic acid sequence as set forth in SEQ ID NO: 38; and/or,
(ii) a mitochondrial DNA (mtDNA) molecule associated with UV exposure, wherein the mtDNA molecule comprises the nucleic acid sequence as set forth in SEQ ID NO: 19; and
(d) comparing the result of the detection step with a reference value.

2. The method of claim 1, wherein the reference value is a control or a value detected in another skin care product.

3. The method of claim 1 or 2, wherein the test sample is exposed to at least one sub-lethal dose of UVR prior to the step of detecting the presence of the fusion transcript or the mtDNA molecule, preferably the test sample is exposed to a series of repetitive sub-lethal doses of UVR.

4. The method of claim 3, wherein the series of repetitive sub-lethal doses of UVR comprises exposing the test sample to daily doses of UVR, preferably the UVR is from a solar-simulated UVR source and the UVR comprises UVA, UVB, or UVA/UVB.

5. An isolated mitochondrial fusion transcript associated with UV exposure, wherein the transcript comprises the nucleic acid sequence as set forth in SEQ ID NO: 38.

6. A method of detecting or monitoring ultraviolet radiation (UVR) exposure in a biological sample comprising detecting:
(i) the presence of a mitochondrial fusion transcript in the biological sample, wherein the transcript comprises the nucleic acid sequence as set forth in SEQ ID NO: 38 and is associated with UVR exposure; and/or,
(ii) the presence of a mtDNA molecule in the biological sample, wherein the mtDNA molecule comprises the nucleic acid sequence as set forth in SEQ ID NO: 19 and is associated with UVR exposure.

7. The method of claim 6, wherein the biological sample is a skin sample taken from a subject or a tissue culture sample.

8. The method of claim 7, wherein the skin sample is taken from an epidermis layer of the subject.

9. The method of claim 6, further comprising exposing the biological sample to at least one sub-lethal dose, and preferably a series of sub-lethal doses, of ultraviolet radiation (UVR) prior to the step of detecting the presence of the fusion transcript and/or mtDNA.

10. The method of claim 9, wherein the series of repetitive sub-lethal doses comprises exposing the biological sample to daily doses of UVR, preferably the UVR is from a solar-simulated UVR source and the UVR comprises UVA, UVB, or UVA/UVB.

11. A method for determining cumulative UV exposure in a subject, comprising:
- detecting the presence of:
(i) a mitochondrial fusion transcript associated with UV exposure and comprising the nucleic acid sequence as set forth in SEQ ID NO: 38; and/or,
(ii) a mtDNA molecule associated with UV exposure and comprising the nucleic acid sequence as set forth in SEQ ID NO: 19
in biological samples obtained from the subject over a period of time; and,
- comparing the presence of the transcript and/or the mtDNA in one of the biological samples with the presence of the transcript and/or the mtDNA in a biological sample obtained from the subject at an earlier time point or with the presence of the transcript and/or the mtDNA in a biological reference sample or a control transcript and/or mtDNA.

12. The method of claim 11, wherein the biological reference sample comprises rarely sun exposed skin samples, occasionally sun exposed skin samples, usually sun exposed skin samples or blood.

13. The method of claim 11 or 12, wherein the biological samples are from rarely sun exposed, occasionally sun exposed, or usually sun exposed skin.

14. Use of a fusion transcript and/or a mtDNA molecule for detecting UV exposure or as a biomarker for UV exposure, wherein the fusion transcript comprises the nucleic acid sequence as set forth in SEQ ID NO: 38 and the mtDNA molecule comprises the nucleic acid sequence as set forth in SEQ ID NO: 19.


SEQUENCE LISTING

<110>  MDNA Life Sciences Inc.

<120>  UV ASSOCIATED mtDNA FUSION TRANSCRIPTS AND METHODS AND USES
       THEREOF

<130>  P6042695EP1

<140>  EP
<141>

<150>  US 61/309,216
<151>  2010-03-01

<150>  PCT/CA2011/050120
<151>  2011-03-01

<150>  EP2542680
<151>  2011-03-01

<160>  45

<170>  PatentIn version 3.3

<210>  1
<211>  16569
<212>  DNA
<213>  Human


<220>
<221>  misc_feature
<222>  (3107)..(3107)
<223>  n is a, c, g, or t

<400>  1
   gatcacaggt ctatcaccct attaaccact cacgggagct ctccatgcat ttggtatttt      60
   cgtctggggg gtatgcacgc gatagcattg cgagacgctg gagccggagc accctatgtc     120
   gcagtatctg tctttgattc ctgcctcatc ctattattta tcgcacctac gttcaatatt     180
   acaggcgaac atacttacta aagtgtgtta attaattaat gcttgtagga cataataata     240
   acaattgaat gtctgcacag ccactttcca cacagacatc ataacaaaaa atttccacca     300
   aaccccccct cccccgcttc tggccacagc acttaaacac atctctgcca aaccccaaaa     360
   acaaagaacc ctaacaccag cctaaccaga tttcaaattt tatcttttgg cggtatgcac     420
   ttttaacagt caccccccaa ctaacacatt attttcccct cccactccca tactactaat     480
   ctcatcaata caacccccgc ccatcctacc cagcacacac acaccgctgc taaccccata     540
   ccccgaacca accaaacccc aaagacaccc cccacagttt atgtagctta cctcctcaaa     600
   gcaatacact gaaaatgttt agacgggctc acatcacccc ataaacaaat aggtttggtc     660
   ctagcctttc tattagctct tagtaagatt acacatgcaa gcatccccgt tccagtgagt     720
   tcaccctcta aatcaccacg atcaaaagga acaagcatca agcacgcagc aatgcagctc     780
   aaaacgctta gcctagccac acccccacgg gaaacagcag tgattaacct ttagcaataa 840
   acgaaagttt aactaagcta tactaacccc agggttggtc aatttcgtgc cagccaccgc 900
   ggtcacacga ttaacccaag tcaatagaag ccggcgtaaa gagtgtttta gatcaccccc 960
   tccccaataa agctaaaact cacctgagtt gtaaaaaact ccagttgaca caaaatagac 1020
   tacgaaagtg gctttaacat atctgaacac acaatagcta agacccaaac tgggattaga 1080
   taccccacta tgcttagccc taaacctcaa cagttaaatc aacaaaactg ctcgccagaa 1140
   cactacgagc cacagcttaa aactcaaagg acctggcggt gcttcatatc cctctagagg 1200
   agcctgttct gtaatcgata aaccccgatc aacctcacca cctcttgctc agcctatata 1260
   ccgccatctt cagcaaaccc tgatgaaggc tacaaagtaa gcgcaagtac ccacgtaaag 1320
   acgttaggtc aaggtgtagc ccatgaggtg gcaagaaatg ggctacattt tctaccccag 1380
   aaaactacga tagcccttat gaaacttaag ggtcgaaggt ggatttagca gtaaactaag 1440
   agtagagtgc ttagttgaac agggccctga agcgcgtaca caccgcccgt caccctcctc 1500
   aagtatactt caaaggacat ttaactaaaa cccctacgca tttatataga ggagacaagt 1560
   cgtaacatgg taagtgtact ggaaagtgca cttggacgaa ccagagtgta gcttaacaca 1620
   aagcacccaa cttacactta ggagatttca acttaacttg accgctctga gctaaaccta 1680
   gccccaaacc cactccacct tactaccaga caaccttagc caaaccattt acccaaataa 1740
   agtataggcg atagaaattg aaacctggcg caatagatat agtaccgcaa gggaaagatg 1800
   aaaaattata accaagcata atatagcaag gactaacccc tataccttct gcataatgaa 1860
   ttaactagaa ataactttgc aaggagagcc aaagctaaga cccccgaaac cagacgagct 1920
   acctaagaac agctaaaaga gcacacccgt ctatgtagca aaatagtggg aagatttata 1980
   ggtagaggcg acaaacctac cgagcctggt gatagctggt tgtccaagat agaatcttag 2040
   ttcaacttta aatttgccca cagaaccctc taaatcccct tgtaaattta actgttagtc 2100
   caaagaggaa cagctctttg gacactagga aaaaaccttg tagagagagt aaaaaattta 2160
   acacccatag taggcctaaa agcagccacc aattaagaaa gcgttcaagc tcaacaccca 2220
   ctacctaaaa aatcccaaac atataactga actcctcaca cccaattgga ccaatctatc 2280
   accctataga agaactaatg ttagtataag taacatgaaa acattctcct ccgcataagc 2340
   ctgcgtcaga ttaaaacact gaactgacaa ttaacagccc aatatctaca atcaaccaac 2400
   aagtcattat taccctcact gtcaacccaa cacaggcatg ctcataagga aaggttaaaa 2460
   aaagtaaaag gaactcggca aatcttaccc cgcctgttta ccaaaaacat cacctctagc 2520
   atcaccagta ttagaggcac cgcctgccca gtgacacatg tttaacggcc gcggtaccct 2580
   aaccgtgcaa aggtagcata atcacttgtt ccttaaatag ggacctgtat gaatggctcc 2640
   acgagggttc agctgtctct tacttttaac cagtgaaatt gacctgcccg tgaagaggcg 2700
   ggcataacac agcaagacga gaagacccta tggagcttta atttattaat gcaaacagta 2760
   cctaacaaac ccacaggtcc taaactacca aacctgcatt aaaaatttcg gttggggcga 2820
   cctcggagca gaacccaacc tccgagcagt acatgctaag acttcaccag tcaaagcgaa 2880
   ctactatact caattgatcc aataacttga ccaacggaac aagttaccct agggataaca 2940
   gcgcaatcct attctagagt ccatatcaac aatagggttt acgacctcga tgttggatca 3000
   ggacatcccg atggtgcagc cgctattaaa ggttcgtttg ttcaacgatt aaagtcctac 3060
   gtgatctgag ttcagaccgg agtaatccag gtcggtttct atctacnttc aaattcctcc 3120
   ctgtacgaaa ggacaagaga aataaggcct acttcacaaa gcgccttccc ccgtaaatga 3180
   tatcatctca acttagtatt atacccacac ccacccaaga acagggtttg ttaagatggc 3240
   agagcccggt aatcgcataa aacttaaaac tttacagtca gaggttcaat tcctcttctt 3300
   aacaacatac ccatggccaa cctcctactc ctcattgtac ccattctaat cgcaatggca 3360
   ttcctaatgc ttaccgaacg aaaaattcta ggctatatac aactacgcaa aggccccaac 3420
   gttgtaggcc cctacgggct actacaaccc ttcgctgacg ccataaaact cttcaccaaa 3480
   gagcccctaa aacccgccac atctaccatc accctctaca tcaccgcccc gaccttagct 3540
   ctcaccatcg ctcttctact atgaaccccc ctccccatac ccaaccccct ggtcaacctc 3600
   aacctaggcc tcctatttat tctagccacc tctagcctag ccgtttactc aatcctctga 3660
   tcagggtgag catcaaactc aaactacgcc ctgatcggcg cactgcgagc agtagcccaa 3720
   acaatctcat atgaagtcac cctagccatc attctactat caacattact aataagtggc 3780
   tcctttaacc tctccaccct tatcacaaca caagaacacc tctgattact cctgccatca 3840
   tgacccttgg ccataatatg atttatctcc acactagcag agaccaaccg aacccccttc 3900
   gaccttgccg aaggggagtc cgaactagtc tcaggcttca acatcgaata cgccgcaggc 3960
   cccttcgccc tattcttcat agccgaatac acaaacatta ttataataaa caccctcacc 4020
   actacaatct tcctaggaac aacatatgac gcactctccc ctgaactcta cacaacatat 4080
   tttgtcacca agaccctact tctaacctcc ctgttcttat gaattcgaac agcatacccc 4140
   cgattccgct acgaccaact catacacctc ctatgaaaaa acttcctacc actcacccta 4200
   gcattactta tatgatatgt ctccataccc attacaatct ccagcattcc ccctcaaacc 4260
   taagaaatat gtctgataaa agagttactt tgatagagta aataatagga gcttaaaccc 4320
   ccttatttct aggactatga gaatcgaacc catccctgag aatccaaaat tctccgtgcc 4380
   acctatcaca ccccatccta aagtaaggtc agctaaataa gctatcgggc ccataccccg 4440
   aaaatgttgg ttataccctt cccgtactaa ttaatcccct ggcccaaccc gtcatctact 4500
   ctaccatctt tgcaggcaca ctcatcacag cgctaagctc gcactgattt tttacctgag 4560
   taggcctaga aataaacatg ctagctttta ttccagttct aaccaaaaaa ataaaccctc 4620
   gttccacaga agctgccatc aagtatttcc tcacgcaagc aaccgcatcc ataatccttc 4680
   taatagctat cctcttcaac aatatactct ccggacaatg aaccataacc aatactacca 4740
   atcaatactc atcattaata atcataatag ctatagcaat aaaactagga atagccccct 4800
   ttcacttctg agtcccagag gttacccaag gcacccctct gacatccggc ctgcttcttc 4860
   tcacatgaca aaaactagcc cccatctcaa tcatatacca aatctctccc tcactaaacg 4920
   taagccttct cctcactctc tcaatcttat ccatcatagc aggcagttga ggtggattaa 4980
   accaaaccca gctacgcaaa atcttagcat actcctcaat tacccacata ggatgaataa 5040
   tagcagttct accgtacaac cctaacataa ccattcttaa tttaactatt tatattatcc 5100
   taactactac cgcattccta ctactcaact taaactccag caccacgacc ctactactat 5160
   ctcgcacctg aaacaagcta acatgactaa cacccttaat tccatccacc ctcctctccc 5220
   taggaggcct gcccccgcta accggctttt tgcccaaatg ggccattatc gaagaattca 5280
   caaaaaacaa tagcctcatc atccccacca tcatagccac catcaccctc cttaacctct 5340
   acttctacct acgcctaatc tactccacct caatcacact actccccata tctaacaacg 5400
   taaaaataaa atgacagttt gaacatacaa aacccacccc attcctcccc acactcatcg 5460
   cccttaccac gctactccta cctatctccc cttttatact aataatctta tagaaattta 5520
   ggttaaatac agaccaagag ccttcaaagc cctcagtaag ttgcaatact taatttctgt 5580
   aacagctaag gactgcaaaa ccccactctg catcaactga acgcaaatca gccactttaa 5640
   ttaagctaag cccttactag accaatggga cttaaaccca caaacactta gttaacagct 5700
   aagcacccta atcaactggc ttcaatctac ttctcccgcc gccgggaaaa aaggcgggag 5760
   aagccccggc aggtttgaag ctgcttcttc gaatttgcaa ttcaatatga aaatcacctc 5820
   ggagctggta aaaagaggcc taacccctgt ctttagattt acagtccaat gcttcactca 5880
   gccattttac ctcaccccca ctgatgttcg ccgaccgttg actattctct acaaaccaca 5940
   aagacattgg aacactatac ctattattcg gcgcatgagc tggagtccta ggcacagctc 6000
   taagcctcct tattcgagcc gagctgggcc agccaggcaa ccttctaggt aacgaccaca 6060
   tctacaacgt tatcgtcaca gcccatgcat ttgtaataat cttcttcata gtaataccca 6120
   tcataatcgg aggctttggc aactgactag ttcccctaat aatcggtgcc cccgatatgg 6180
   cgtttccccg cataaacaac ataagcttct gactcttacc tccctctctc ctactcctgc 6240
   tcgcatctgc tatagtggag gccggagcag gaacaggttg aacagtctac cctcccttag 6300
   cagggaacta ctcccaccct ggagcctccg tagacctaac catcttctcc ttacacctag 6360
   caggtgtctc ctctatctta ggggccatca atttcatcac aacaattatc aatataaaac 6420
   cccctgccat aacccaatac caaacgcccc tcttcgtctg atccgtccta atcacagcag 6480
   tcctacttct cctatctctc ccagtcctag ctgctggcat cactatacta ctaacagacc 6540
   gcaacctcaa caccaccttc ttcgaccccg ccggaggagg agaccccatt ctataccaac 6600
   acctattctg atttttcggt caccctgaag tttatattct tatcctacca ggcttcggaa 6660
   taatctccca tattgtaact tactactccg gaaaaaaaga accatttgga tacataggta 6720
   tggtctgagc tatgatatca attggcttcc tagggtttat cgtgtgagca caccatatat 6780
   ttacagtagg aatagacgta gacacacgag catatttcac ctccgctacc ataatcatcg 6840
   ctatccccac cggcgtcaaa gtatttagct gactcgccac actccacgga agcaatatga 6900
   aatgatctgc tgcagtgctc tgagccctag gattcatctt tcttttcacc gtaggtggcc 6960
   tgactggcat tgtattagca aactcatcac tagacatcgt actacacgac acgtactacg 7020
   ttgtagccca cttccactat gtcctatcaa taggagctgt atttgccatc ataggaggct 7080
   tcattcactg atttccccta ttctcaggct acaccctaga ccaaacctac gccaaaatcc 7140
   atttcactat catattcatc ggcgtaaatc taactttctt cccacaacac tttctcggcc 7200
   tatccggaat gccccgacgt tactcggact accccgatgc atacaccaca tgaaacatcc 7260
   tatcatctgt aggctcattc atttctctaa cagcagtaat attaataatt ttcatgattt 7320
   gagaagcctt cgcttcgaag cgaaaagtcc taatagtaga agaaccctcc ataaacctgg 7380
   agtgactata tggatgcccc ccaccctacc acacattcga agaacccgta tacataaaat 7440
   ctagacaaaa aaggaaggaa tcgaaccccc caaagctggt ttcaagccaa ccccatggcc 7500
   tccatgactt tttcaaaaag gtattagaaa aaccatttca taactttgtc aaagttaaat 7560
   tataggctaa atcctatata tcttaatggc acatgcagcg caagtaggtc tacaagacgc 7620
   tacttcccct atcatagaag agcttatcac ctttcatgat cacgccctca taatcatttt 7680
   ccttatctgc ttcctagtcc tgtatgccct tttcctaaca ctcacaacaa aactaactaa 7740
   tactaacatc tcagacgctc aggaaataga aaccgtctga actatcctgc ccgccatcat 7800
   cctagtcctc atcgccctcc catccctacg catcctttac ataacagacg aggtcaacga 7860
   tccctccctt accatcaaat caattggcca ccaatggtac tgaacctacg agtacaccga 7920
   ctacggcgga ctaatcttca actcctacat acttccccca ttattcctag aaccaggcga 7980
   cctgcgactc cttgacgttg acaatcgagt agtactcccg attgaagccc ccattcgtat 8040
   aataattaca tcacaagacg tcttgcactc atgagctgtc cccacattag gcttaaaaac 8100
   agatgcaatt cccggacgtc taaaccaaac cactttcacc gctacacgac cgggggtata 8160
   ctacggtcaa tgctctgaaa tctgtggagc aaaccacagt ttcatgccca tcgtcctaga 8220
   attaattccc ctaaaaatct ttgaaatagg gcccgtattt accctatagc accccctcta 8280
   ccccctctag agcccactgt aaagctaact tagcattaac cttttaagtt aaagattaag 8340
   agaaccaaca cctctttaca gtgaaatgcc ccaactaaat actaccgtat ggcccaccat 8400
   aattaccccc atactcctta cactattcct catcacccaa ctaaaaatat taaacacaaa 8460
   ctaccaccta cctccctcac caaagcccat aaaaataaaa aattataaca aaccctgaga 8520
   accaaaatga acgaaaatct gttcgcttca ttcattgccc ccacaatcct aggcctaccc 8580
   gccgcagtac tgatcattct atttccccct ctattgatcc ccacctccaa atatctcatc 8640
   aacaaccgac taatcaccac ccaacaatga ctaatcaaac taacctcaaa acaaatgata 8700
   accatacaca acactaaagg acgaacctga tctcttatac tagtatcctt aatcattttt 8760
   attgccacaa ctaacctcct cggactcctg cctcactcat ttacaccaac cacccaacta 8820
   tctataaacc tagccatggc catcccctta tgagcgggca cagtgattat aggctttcgc 8880
   tctaagatta aaaatgccct agcccacttc ttaccacaag gcacacctac accccttatc 8940
   cccatactag ttattatcga aaccatcagc ctactcattc aaccaatagc cctggccgta 9000
   cgcctaaccg ctaacattac tgcaggccac ctactcatgc acctaattgg aagcgccacc 9060
   ctagcaatat caaccattaa ccttccctct acacttatca tcttcacaat tctaattcta 9120
   ctgactatcc tagaaatcgc tgtcgcctta atccaagcct acgttttcac acttctagta 9180
   agcctctacc tgcacgacaa cacataatga cccaccaatc acatgcctat catatagtaa 9240
   aacccagccc atgaccccta acaggggccc tctcagccct cctaatgacc tccggcctag 9300
   ccatgtgatt tcacttccac tccataacgc tcctcatact aggcctacta accaacacac 9360
   taaccatata ccaatgatgg cgcgatgtaa cacgagaaag cacataccaa ggccaccaca 9420
   caccacctgt ccaaaaaggc cttcgatacg ggataatcct atttattacc tcagaagttt 9480
   ttttcttcgc aggatttttc tgagcctttt accactccag cctagcccct accccccaat 9540
   taggagggca ctggccccca acaggcatca ccccgctaaa tcccctagaa gtcccactcc 9600
   taaacacatc cgtattactc gcatcaggag tatcaatcac ctgagctcac catagtctaa 9660
   tagaaaacaa ccgaaaccaa ataattcaag cactgcttat tacaatttta ctgggtctct 9720
   attttaccct cctacaagcc tcagagtact tcgagtctcc cttcaccatt tccgacggca 9780
   tctacggctc aacatttttt gtagccacag gcttccacgg acttcacgtc attattggct 9840
   caactttcct cactatctgc ttcatccgcc aactaatatt tcactttaca tccaaacatc 9900
   actttggctt cgaagccgcc gcctgatact ggcattttgt agatgtggtt tgactatttc 9960
   tgtatgtctc catctattga tgagggtctt actcttttag tataaatagt accgttaact 10020
   tccaattaac tagttttgac aacattcaaa aaagagtaat aaacttcgcc ttaattttaa 10080
   taatcaacac cctcctagcc ttactactaa taattattac attttgacta ccacaactca 10140
   acggctacat agaaaaatcc accccttacg agtgcggctt cgaccctata tcccccgccc 10200
   gcgtcccttt ctccataaaa ttcttcttag tagctattac cttcttatta tttgatctag 10260
   aaattgccct ccttttaccc ctaccatgag ccctacaaac aactaacctg ccactaatag 10320
   ttatgtcatc cctcttatta atcatcatcc tagccctaag tctggcctat gagtgactac 10380
   aaaaaggatt agactgaacc gaattggtat atagtttaaa caaaacgaat gatttcgact 10440
   cattaaatta tgataatcat atttaccaaa tgcccctcat ttacataaat attatactag 10500
   catttaccat ctcacttcta ggaatactag tatatcgctc acacctcata tcctccctac 10560
   tatgcctaga aggaataata ctatcgctgt tcattatagc tactctcata accctcaaca 10620
   cccactccct cttagccaat attgtgccta ttgccatact agtctttgcc gcctgcgaag 10680
   cagcggtggg cctagcccta ctagtctcaa tctccaacac atatggccta gactacgtac 10740
   ataacctaaa cctactccaa tgctaaaact aatcgtccca acaattatat tactaccact 10800
   gacatgactt tccaaaaaac acataatttg aatcaacaca accacccaca gcctaattat 10860
   tagcatcatc cctctactat tttttaacca aatcaacaac aacctattta gctgttcccc 10920
   aaccttttcc tccgaccccc taacaacccc cctcctaata ctaactacct gactcctacc 10980
   cctcacaatc atggcaagcc aacgccactt atccagtgaa ccactatcac gaaaaaaact 11040
   ctacctctct atactaatct ccctacaaat ctccttaatt ataacattca cagccacaga 11100
   actaatcata ttttatatct tcttcgaaac cacacttatc cccaccttgg ctatcatcac 11160
   ccgatgaggc aaccagccag aacgcctgaa cgcaggcaca tacttcctat tctacaccct 11220
   agtaggctcc cttcccctac tcatcgcact aatttacact cacaacaccc taggctcact 11280
   aaacattcta ctactcactc tcactgccca agaactatca aactcctgag ccaacaactt 11340
   aatatgacta gcttacacaa tagcttttat agtaaagata cctctttacg gactccactt 11400
   atgactccct aaagcccatg tcgaagcccc catcgctggg tcaatagtac ttgccgcagt 11460
   actcttaaaa ctaggcggct atggtataat acgcctcaca ctcattctca accccctgac 11520
   aaaacacata gcctacccct tccttgtact atccctatga ggcataatta taacaagctc 11580
   catctgccta cgacaaacag acctaaaatc gctcattgca tactcttcaa tcagccacat 11640
   agccctcgta gtaacagcca ttctcatcca aaccccctga agcttcaccg gcgcagtcat 11700
   tctcataatc gcccacgggc ttacatcctc attactattc tgcctagcaa actcaaacta 11760
   cgaacgcact cacagtcgca tcataatcct ctctcaagga cttcaaactc tactcccact 11820
   aatagctttt tgatgacttc tagcaagcct cgctaacctc gccttacccc ccactattaa 11880
   cctactggga gaactctctg tgctagtaac cacgttctcc tgatcaaata tcactctcct 11940
   acttacagga ctcaacatac tagtcacagc cctatactcc ctctacatat ttaccacaac 12000
   acaatggggc tcactcaccc accacattaa caacataaaa ccctcattca cacgagaaaa 12060
   caccctcatg ttcatacacc tatcccccat tctcctccta tccctcaacc ccgacatcat 12120
   taccgggttt tcctcttgta aatatagttt aaccaaaaca tcagattgtg aatctgacaa 12180
   cagaggctta cgacccctta tttaccgaga aagctcacaa gaactgctaa ctcatgcccc 12240
   catgtctaac aacatggctt tctcaacttt taaaggataa cagctatcca ttggtcttag 12300
   gccccaaaaa ttttggtgca actccaaata aaagtaataa ccatgcacac tactataacc 12360
   accctaaccc tgacttccct aattcccccc atccttacca ccctcgttaa ccctaacaaa 12420
   aaaaactcat acccccatta tgtaaaatcc attgtcgcat ccacctttat tatcagtctc 12480
   ttccccacaa caatattcat gtgcctagac caagaagtta ttatctcgaa ctgacactga 12540
   gccacaaccc aaacaaccca gctctcccta agcttcaaac tagactactt ctccataata 12600
   ttcatccctg tagcattgtt cgttacatgg tccatcatag aattctcact gtgatatata 12660
   aactcagacc caaacattaa tcagttcttc aaatatctac tcatcttcct aattaccata 12720
   ctaatcttag ttaccgctaa caacctattc caactgttca tcggctgaga gggcgtagga 12780
   attatatcct tcttgctcat cagttgatga tacgcccgag cagatgccaa cacagcagcc 12840
   attcaagcaa tcctatacaa ccgtatcggc gatatcggtt tcatcctcgc cttagcatga 12900
   tttatcctac actccaactc atgagaccca caacaaatag cccttctaaa cgctaatcca 12960
   agcctcaccc cactactagg cctcctccta gcagcagcag gcaaatcagc ccaattaggt 13020
   ctccacccct gactcccctc agccatagaa ggccccaccc cagtctcagc cctactccac 13080
   tcaagcacta tagttgtagc aggaatcttc ttactcatcc gcttccaccc cctagcagaa 13140
   aatagcccac taatccaaac tctaacacta tgcttaggcg ctatcaccac tctgttcgca 13200
   gcagtctgcg cccttacaca aaatgacatc aaaaaaatcg tagccttctc cacttcaagt 13260
   caactaggac tcataatagt tacaatcggc atcaaccaac cacacctagc attcctgcac 13320
   atctgtaccc acgccttctt caaagccata ctatttatgt gctccgggtc catcatccac 13380
   aaccttaaca atgaacaaga tattcgaaaa ataggaggac tactcaaaac catacctctc 13440
   acttcaacct ccctcaccat tggcagccta gcattagcag gaataccttt cctcacaggt 13500
   ttctactcca aagaccacat catcgaaacc gcaaacatat catacacaaa cgcctgagcc 13560
   ctatctatta ctctcatcgc tacctccctg acaagcgcct atagcactcg aataattctt 13620
   ctcaccctaa caggtcaacc tcgcttcccc acccttacta acattaacga aaataacccc 13680
   accctactaa accccattaa acgcctggca gccggaagcc tattcgcagg atttctcatt 13740
   actaacaaca tttcccccgc atcccccttc caaacaacaa tccccctcta cctaaaactc 13800
   acagccctcg ctgtcacttt cctaggactt ctaacagccc tagacctcaa ctacctaacc 13860
   aacaaactta aaataaaatc cccactatgc acattttatt tctccaacat actcggattc 13920
   taccctagca tcacacaccg cacaatcccc tatctaggcc ttcttacgag ccaaaacctg 13980
   cccctactcc tcctagacct aacctgacta gaaaagctat tacctaaaac aatttcacag 14040
   caccaaatct ccacctccat catcacctca acccaaaaag gcataattaa actttacttc 14100
   ctctctttct tcttcccact catcctaacc ctactcctaa tcacataacc tattcccccg 14160
   agcaatctca attacaatat atacaccaac aaacaatgtt caaccagtaa ctactactaa 14220
   tcaacgccca taatcataca aagcccccgc accaatagga tcctcccgaa tcaaccctga 14280
   cccctctcct tcataaatta ttcagcttcc tacactatta aagtttacca caaccaccac 14340
   cccatcatac tctttcaccc acagcaccaa tcctacctcc atcgctaacc ccactaaaac 14400
   actcaccaag acctcaaccc ctgaccccca tgcctcagga tactcctcaa tagccatcgc 14460
   tgtagtatat ccaaagacaa ccatcattcc ccctaaataa attaaaaaaa ctattaaacc 14520
   catataacct cccccaaaat tcagaataat aacacacccg accacaccgc taacaatcaa 14580
   tactaaaccc ccataaatag gagaaggctt agaagaaaac cccacaaacc ccattactaa 14640
   acccacactc aacagaaaca aagcatacat cattattctc gcacggacta caaccacgac 14700
   caatgatatg aaaaaccatc gttgtatttc aactacaaga acaccaatga ccccaatacg 14760
   caaaactaac cccctaataa aattaattaa ccactcattc atcgacctcc ccaccccatc 14820
   caacatctcc gcatgatgaa acttcggctc actccttggc gcctgcctga tcctccaaat 14880
   caccacagga ctattcctag ccatgcacta ctcaccagac gcctcaaccg ccttttcatc 14940
   aatcgcccac atcactcgag acgtaaatta tggctgaatc atccgctacc ttcacgccaa 15000
   tggcgcctca atattcttta tctgcctctt cctacacatc gggcgaggcc tatattacgg 15060
   atcatttctc tactcagaaa cctgaaacat cggcattatc ctcctgcttg caactatagc 15120
   aacagccttc ataggctatg tcctcccgtg aggccaaata tcattctgag gggccacagt 15180
   aattacaaac ttactatccg ccatcccata cattgggaca gacctagttc aatgaatctg 15240
   aggaggctac tcagtagaca gtcccaccct cacacgattc tttacctttc acttcatctt 15300
   gcccttcatt attgcagccc tagcaacact ccacctccta ttcttgcacg aaacgggatc 15360
   aaacaacccc ctaggaatca cctcccattc cgataaaatc accttccacc cttactacac 15420
   aatcaaagac gccctcggct tacttctctt ccttctctcc ttaatgacat taacactatt 15480
   ctcaccagac ctcctaggcg acccagacaa ttatacccta gccaacccct taaacacccc 15540
   tccccacatc aagcccgaat gatatttcct attcgcctac acaattctcc gatccgtccc 15600
   taacaaacta ggaggcgtcc ttgccctatt actatccatc ctcatcctag caataatccc 15660
   catcctccat atatccaaac aacaaagcat aatatttcgc ccactaagcc aatcacttta 15720
   ttgactccta gccgcagacc tcctcattct aacctgaatc ggaggacaac cagtaagcta 15780
   cccttttacc atcattggac aagtagcatc cgtactatac ttcacaacaa tcctaatcct 15840
   aataccaact atctccctaa ttgaaaacaa aatactcaaa tgggcctgtc cttgtagtat 15900
   aaactaatac accagtcttg taaaccggag atgaaaacct ttttccaagg acaaatcaga 15960
   gaaaaagtct ttaactccac cattagcacc caaagctaag attctaattt aaactattct 16020
   ctgttctttc atggggaagc agatttgggt accacccaag tattgactca cccatcaaca 16080
   accgctatgt atttcgtaca ttactgccag ccaccatgaa tattgtacgg taccataaat 16140
   acttgaccac ctgtagtaca taaaaaccca atccacatca aaaccccctc cccatgctta 16200
   caagcaagta cagcaatcaa ccctcaacta tcacacatca actgcaactc caaagccacc 16260
   cctcacccac taggatacca acaaacctac ccacccttaa cagtacatag tacataaagc 16320
   catttaccgt acatagcaca ttacagtcaa atcccttctc gtccccatgg atgacccccc 16380
   tcagataggg gtcccttgac caccatcctc cgtgaaatca atatcccgca caagagtgct 16440
   actctcctcg ctccgggccc ataacacttg ggggtagcta aagtgaactg tatccgacat 16500
   ctggttccta cttcagggtc ataaagccta aatagcccac acgttcccct taaataagac 16560
   atcacgatg 16569

<210> 2
<211> 783
<212> DNA
<213> Artificial

<220>
<223> cDNA

<400> 2
atggcccacc ataattaccc ccatactcct tacactattc ctcatcaccc aactaaaaat 60

attaaacaca aactaccacc tacctccctc accattggca gcctagcatt agcaggaata 120

cctttcctca caggtttcta ctccaaagac cacatcatcg aaaccgcaaa catatcatac 180

acaaacgcct gagccctatc tattactctc atcgctacct ccctgacaag cgcctatagc 240

actcgaataa ttcttctcac cctaacaggt caacctcgct tccccaccct tactaacatt 300

aacgaaaata accccaccct actaaacccc attaaacgcc tggcagccgg aagcctattc 360

gcaggatttc tcattactaa caacatttcc cccgcatccc ccttccaaac aacaatcccc 420

ctctacctaa aactcacagc cctcgctgtc actttcctag gacttctaac agccctagac 480

ctcaactacc taaccaacaa acttaaaata aaatccccac tatgcacatt ttatttctcc 540

aacatactcg gattctaccc tagcatcaca caccgcacaa tcccctatct aggccttctt 600

acgagccaaa acctgcccct actcctccta gacctaacct gactagaaaa gctattacct 660

aaaacaattt cacagcacca aatctccacc tccatcatca cctcaaccca aaaaggcata 720

attaaacttt acttcctctc tttcttcttc ccactcatcc taaccctact cctaatcaca 780

taa 783

<210>  3
<211>  300
<212>  DNA
<213>  Artificial

<220>
<223>  cDNA

<400>  3
atgcccctca tttacataaa tattatacta gcatttacca tctcacttct aggaatacta      60

gtatatcgct cacacctcat atcctcccta ctatgcctag aaggaataat actatcgctg     120

ttcattatag ctactctcat aaccctcaac acccactccc tcttagccaa tattgtgcct     180

attgccatac tagtctttgc cgcctgcgaa gcagcggtgg gcctagccct actagtctca     240

atctccaaca catatggcct agactacgta cataacctaa ccctactcct aatcacataa     300


<210>  4
<211>  781
<212>  DNA
<213>  Artificial

<220>
<223>  cDNA

<400>  4
atggcacatg cagcgcaagt aggtctacaa gacgctactt cccctatcat agaagagctt      60

atcacctttc atgatcacgc cctcataatc attttcctta tctgcttcct agtcctgtat     120

gcccttttcc taacactcac aacaaaacta actaatacta acatctcaga cgctcaggaa     180

atagaaaccg tctgaactat cctgcccgcc atcatcctag tcctcatcgc cctcccatcc     240

ctacgcatcc tttacataac agacgaggtc aacgatccct cccttaccat caaatcaatt     300

ggccaccaat ggtactgaac ctacgagtac accgactacg gcggactaat cttcaactcc     360

tacatacttc ccccattatt cctagaacca ggcgacccag acaattatac cctagccaac     420

cccttaaaca cccctcccca catcaagccc gaatgatatt tcctattcgc ctacacaatt     480

ctccgatccg tccctaacaa actaggaggc gtccttgccc tattactatc catcctcatc     540

ctagcaataa tccccatcct ccatatatcc aaacaacaaa gcataatatt tcgcccacta     600

agccaatcac tttattgact cctagccgca gacctcctca ttctaacctg aatcggagga     660

caaccagtaa gctacccttt taccatcatt ggacaagtag catccgtact atacttcaca     720

acaatcctaa tcctaatacc aactatctcc ctaattgaaa acaaaatact caaatgggcc     780

t                                                                     781


<210>  5
<211>  565
<212>  DNA
<213>  Artificial

<220>
<223>  cDNA

<400>  5
atggcacatg cagcgcaagt aggtctacaa gacgctactt cccctatcat agaagagctt      60

atcacctttc atgatcacgc cctcataatc attttcctta tctgcttcct agtcctgtat     120

gcccttttcc taacactcac aacaaaacta actaatacta acatctcaga cgctcaggaa     180

atagaaaccg tctgaactat cctgcccgcc atcatcctag tcctcatcgc cctcccatcc     240

ctacgcatcc tttacataac agacgaggtc aacgatccct cccttaccat caaatcaatt     300

ggccaccaat ggtactgaac ctacgagtac accgactacg gcggactaat cttcaactcc     360

tacatacttc ccccattatt cctagaacca ggcgacctgc gactcctagc cgcagacctc     420

ctcattctaa cctgaatcgg aggacaacca gtaagctacc cttttaccat cattggacaa     480

gtagcatccg tactatactt cacaacaatc ctaatcctaa taccaactat ctccctaatt     540

gaaaacaaaa tactcaaatg ggcct                                           565


<210>  6
<211>  1174
<212>  DNA
<213>  Artificial

<220>
<223>  cDNA

<400>  6
atggcacatg cagcgcaagt aggtctacaa gacgctactt cccctatcat agaagagctt      60

atcacctttc atgatcacgc cctcataatc attttcctta tctgcttcct agtcctgtat     120

gcccttttcc taacactcac aacaaaacta actaatacta acatctcaga cgctcaggaa     180

atagaaaccg tctgaactat cctgcccgcc atcatcctag tcctcatcgc cctcccatcc     240

ctacgcatcc tttacataac agacgaggtc aacgatccct cccttaccat caaatcaatt     300

ggccaccaat ggtactgaac ctacgagtac accgactacg gcggactaat cttcaactcc     360

tacatacttc ccccattatt cctagaacca ggcgacctgc gactccttga cgttgacaat     420

cgagtagtac tcccgattga agcccccatt cgtataataa ttacatcaca agacgtcttg     480

cactcatgag ctgtccccac attaggctta aaaacagatg caattcccgg acgtctaaac     540

caaaccactt tcaccgctac acgaccgggg gtatactacg gtcaatgctc tgaaatctgt     600

ggagcaaacc acagtttcat gcccatattc ttgcacgaaa cgggatcaaa caacccccta     660

ggaatcacct cccattccga taaaatcacc ttccaccctt actacacaat caaagacgcc     720

ctcggcttac ttctcttcct tctctcctta atgacattaa cactattctc accagacctc     780

ctaggcgacc cagacaatta taccctagcc aaccccttaa acacccctcc ccacatcaag     840

cccgaatgat atttcctatt cgcctacaca attctccgat ccgtccctaa caaactagga     900

ggcgtccttg ccctattact atccatcctc atcctagcaa taatccccat cctccatata 960

tccaaacaac aaagcataat atttcgccca ctaagccaat cactttattg actcctagcc 1020

gcagacctcc tcattctaac ctgaatcgga ggacaaccag taagctaccc ttttaccatc 1080

attggacaag tagcatccgt actatacttc acaacaatcc taatcctaat accaactatc 1140

tccctaattg aaaacaaaat actcaaatgg gcct 1174

<210> 7
<211> 1294
<212> DNA
<213> Artificial

<220>
<223> cDNA

<400> 7
atgaacgaaa atctgttcgc ttcattcatt gcccccacaa tcctaggcct acccgccgca 60

gtactgatca ttctatttcc ccctctattg atccccacct ccaaatatct catcaacaac 120

cgactaatca ccacccaaca atgactaatc aaactaacct caaaacaaat gataaccata 180

cacaacacta aaggacgaac ctgatctctt atactagtat ccttaatcat ttttattgcc 240

acaactaacc tcctcggact cctgcctcac tcatttacac caaccaccca actatctata 300

aacctagcca tgcactactc accagacgcc tcaaccgcct tttcatcaat cgcccacatc 360

actcgagacg taaattatgg ctgaatcatc cgctaccttc acgccaatgg cgcctcaata 420

ttctttatct gcctcttcct acacatcggg cgaggcctat attacggatc atttctctac 480

tcagaaacct gaaacatcgg cattatcctc ctgcttgcaa ctatagcaac agccttcata 540

ggctatgtcc tcccgtgagg ccaaatatca ttctgagggg ccacagtaat tacaaactta 600

ctatccgcca tcccatacat tgggacagac ctagttcaat gaatctgagg aggctactca 660

gtagacagtc ccaccctcac acgattcttt acctttcact tcatcttgcc cttcattatt 720

gcagccctag caacactcca cctcctattc ttgcacgaaa cgggatcaaa caacccccta 780

ggaatcacct cccattccga taaaatcacc ttccaccctt actacacaat caaagacgcc 840

ctcggcttac ttctcttcct tctctcctta atgacattaa cactattctc accagacctc 900

ctaggcgacc cagacaatta taccctagcc aaccccttaa acacccctcc ccacatcaag 960

cccgaatgat atttcctatt cgcctacaca attctccgat ccgtccctaa caaactagga 1020

ggcgtccttg ccctattact atccatcctc atcctagcaa taatccccat cctccatata 1080

tccaaacaac aaagcataat atttcgccca ctaagccaat cactttattg actcctagcc 1140

gcagacctcc tcattctaac ctgaatcgga ggacaaccag taagctaccc ttttaccatc 1200

attggacaag tagcatccgt actatacttc acaacaatcc taatcctaat accaactatc 1260

tccctaattg aaaacaaaat actcaaatgg gcct 1294

<210> 8
<211> 1228
<212> DNA
<213> Artificial

<220>
<223> cDNA

<400> 8
atgcccctca tttacataaa tattatacta gcatttacca tctcacttct aggaatacta 60

gtatatcgct cacacctcat atcctcccta ctatgcctag aaggaataat actatcgctg 120

ttcattatag ctactctcat aaccctcaac acccactccc tcttagccaa tattgtgcct 180

attgccatac tagtctttgg cgcctgcctg atcctccaaa tcaccacagg actattccta 240

gccatgcact actcaccaga cgcctcaacc gccttttcat caatcgccca catcactcga 300

gacgtaaatt atggctgaat catccgctac cttcacgcca atggcgcctc aatattcttt 360

atctgcctct tcctacacat cgggcgaggc ctatattacg gatcatttct ctactcagaa 420

acctgaaaca tcggcattat cctcctgctt gcaactatag caacagcctt cataggctat 480

gtcctcccgt gaggccaaat atcattctga ggggccacag taattacaaa cttactatcc 540

gccatcccat acattgggac agacctagtt caatgaatct gaggaggcta ctcagtagac 600

agtcccaccc tcacacgatt ctttaccttt cacttcatct tgcccttcat tattgcagcc 660

ctagcaacac tccacctcct attcttgcac gaaacgggat caaacaaccc cctaggaatc 720

acctcccatt ccgataaaat caccttccac ccttactaca caatcaaaga cgccctcggc 780

ttacttctct tccttctctc cttaatgaca ttaacactat tctcaccaga cctcctaggc 840

gacccagaca attataccct agccaacccc ttaaacaccc ctccccacat caagcccgaa 900

tgatatttcc tattcgccta cacaattctc cgatccgtcc ctaacaaact aggaggcgtc 960

cttgccctat tactatccat cctcatccta gcaataatcc ccatcctcca tatatccaaa 1020

caacaaagca taatatttcg cccactaagc caatcacttt attgactcct agccgcagac 1080

ctcctcattc taacctgaat cggaggacaa ccagtaagct acccttttac catcattgga 1140

caagtagcat ccgtactata cttcacaaca atcctaatcc taataccaac tatctcccta 1200

attgaaaaca aaatactcaa atgggcct 1228

<210> 9
<211> 522
<212> DNA
<213> Artificial

<220>
<223> cDNA

<400>  9
atgttcgccg accgttgact attctctaca aaccacaaag acattggaac actataccta      60

ttattcggcg catgagctgg agtcctaggc acagctctaa gcctccttat tcgagccgag     120

ctgggccagc caggcaacct tctaggtaac gaccacatct acaacgttat cgtcacagcc     180

ctcgctgtca ctttcctagg acttctaaca gccctagacc tcaactacct aaccaacaaa     240

cttaaaataa aatccccact atgcacattt tatttctcca acatactcgg attctaccct     300

agcatcacac accgcacaat cccctatcta ggccttctta cgagccaaaa cctgccccta     360

ctcctcctag acctaacctg actagaaaag ctattaccta aaacaatttc acagcaccaa     420

atctccacct ccatcatcac ctcaacccaa aaaggcataa ttaaacttta cttcctctct     480

ttcttcttcc cactcatcct aaccctactc ctaatcacat aa                        522


<210>  10
<211>  582
<212>  DNA
<213>  Artificial

<220>
<223>  cDNA

<400>  10
atgttcgccg accgttgact attctctaca aaccacaaag acattggaac actataccta      60

ttattcggcg catgagctgg agtcctaggc acagctctaa gcctccttat tcgagccgag     120

ctgggccagc caggcaacct tctaggtaac gaccacatct acaacgttat cgtcacagcc     180

catgcatttg taataatctt cttcatagta atacccatca taatcggagg ctttggcaac     240

tgactagttc ccctaataat cggtgccccc gatatggcgt ttccccgcat aaacaacata     300

agcttctgac tcttacctcc ctctctccta ctcctgctcg catctgctat agtggaggcc     360

ggagcaggaa caggttgaac agtctaccct cccttagcag ggaactactc ccaccctgga     420

gccctcctag acctaacctg actagaaaag ctattaccta aaacaatttc acagcaccaa     480

atctccacct ccatcatcac ctcaacccaa aaaggcataa ttaaacttta cttcctctct     540

ttcttcttcc cactcatcct aaccctactc ctaatcacat aa                        582


<210>  11
<211>  2208
<212>  DNA
<213>  Artificial

<220>
<223>  cDNA

<400>  11
atgttcgccg accgttgact attctctaca aaccacaaag acattggaac actataccta      60

ttattcggcg catgagctgg agtcctaggc acagctctaa gcctccttat tcgagccgag     120

ctgggccagc caggcaacct tctaggtaac gaccacatct acaacgttat cgtcacagcc 180

catgcatttg taataatctt cttcatagta atacccatca taatcggagg ctttggcaac 240

tgactagttc ccctaataat cggtgccccc gatatggcgt ttccccgcat aaacaacata 300

agcttctgac tcttacctcc ctctctccta ctcctgctcg catctgctat agtggaggcc 360

ggagcaggaa caggttgaac agtctaccct cccttagcag ggaactactc ccaccctgga 420

gcctccgtag acctaaccat cttctcctta cacctagcag gtgtctcctc tatcttaggg 480

gccatcaatt tcatcacaac aattatcaat ataaaacccc ctgccataac ccaataccaa 540

acgcccctct tcgtctgatc cgtcctaatc acagcagtcc tacttctcct atctctccca 600

gtcctagctg ctggcatcac tatactacta acagaccgca acctcaacac caccttcttc 660

gaccccgccg gaggaggaga ccccattcta taccaacacc tattctgatt tttcggtcac 720

cctgaagttt atattcttat cctaccaggc ttcggaataa tctcccatat tgtaacttac 780

tactccggaa aaaaagaacc atttggatac ataggtatgg tctgagctat gatatcaatt 840

ggcttcctag ggtttatcgt gtgagcacac catatattta cagtaggaat agacgtagac 900

acacgagcat atttcacctc cgctaccata atcatcgcta tccccaccgg cgtcaaagta 960

tttagctgac tcgccacact ccacggaagc aatatgaaat gatctgctgc agtgctctga 1020

gccctaggat tcatctttct tttcaccgta ggtggcctga ctggcattgt attagcaaac 1080

tcatcactag acatcgtact acacgacacg tactacgttg tagcccactt ccactatgtc 1140

ctatcaatag gagctgtatt tgccatcata ggaggcttca ttcactgatt tcccctattc 1200

tcaggctaca ccctagacca aacctacgcc aaaatccatt tcactatcat attcatcggc 1260

gtaaatctaa ctttcttccc acaacacttt ctcggcctat ccggaatgcc ccgacgttac 1320

tcggactacc ccgatgcata caccacatga aacatcctat catctgtagg ctcattcatt 1380

tctctaacag cagtaatatt aataattttc atgatttgag aagccttcgc ttcgaagcga 1440

aaagtcctaa tagtagaaga accctccata aacctggagt gactatatgg atgcccccca 1500

ccctaccaca cattcgaaga acccgtatac ataaaagcag gaataccttt cctcacaggt 1560

ttctactcca aagaccacat catcgaaacc gcaaacatat catacacaaa cgcctgagcc 1620

ctatctatta ctctcatcgc tacctccctg acaagcgcct atagcactcg aataattctt 1680

ctcaccctaa caggtcaacc tcgcttcccc acccttacta acattaacga aaataacccc 1740

accctactaa accccattaa acgcctggca gccggaagcc tattcgcagg atttctcatt 1800

actaacaaca tttcccccgc atcccccttc caaacaacaa tccccctcta cctaaaactc 1860

acagccctcg ctgtcacttt cctaggactt ctaacagccc tagacctcaa ctacctaacc 1920

aacaaactta aaataaaatc cccactatgc acattttatt tctccaacat actcggattc 1980

taccctagca tcacacaccg cacaatcccc tatctaggcc ttcttacgag ccaaaacctg 2040

cccctactcc tcctagacct aacctgacta gaaaagctat tacctaaaac aatttcacag 2100

caccaaatct ccacctccat catcacctca acccaaaaag gcataattaa actttacttc 2160

ctctctttct tcttcccact catcctaacc ctactcctaa tcacataa 2208

<210> 12
<211> 807
<212> DNA
<213> Artificial

<220>
<223> cDNA

<400> 12
atggcacatg cagcgcaagt aggtctacaa gacgctactt cccctatcat agaagagctt 60

atcacctttc atgatcacgc cctcataatc attttcctta tctgcttcct agtcctgtat 120

gcccttttcc taacactcac aacaaaacta actaatacta acatctcaga cgctcaggaa 180

atagaaaccg caaacatatc atacacaaac gcctgagccc tatctattac tctcatcgct 240

acctccctga caagcgccta tagcactcga ataattcttc tcaccctaac aggtcaacct 300

cgcttcccca cccttactaa cattaacgaa aataacccca ccctactaaa ccccattaaa 360

cgcctggcag ccggaagcct attcgcagga tttctcatta ctaacaacat ttcccccgca 420

tcccccttcc aaacaacaat ccccctctac ctaaaactca cagccctcgc tgtcactttc 480

ctaggacttc taacagccct agacctcaac tacctaacca acaaacttaa aataaaatcc 540

ccactatgca cattttattt ctccaacata ctcggattct accctagcat cacacaccgc 600

acaatcccct atctaggcct tcttacgagc caaaacctgc ccctactcct cctagaccta 660

acctgactag aaaagctatt acctaaaaca atttcacagc accaaatctc cacctccatc 720

atcacctcaa cccaaaaagg cataattaaa ctttacttcc tctctttctt cttcccactc 780

atcctaaccc tactcctaat cacataa 807

<210> 13
<211> 786
<212> DNA
<213> Artificial

<220>
<223> cDNA

<400> 13
atggcacatg cagcgcaagt aggtctacaa gacgctactt cccctatcat agaagagctt 60

atcacctttc atgatcacgc cctcataatc attttcctta tctgcttcct agtcctgtat 120

gcccttttcc taacactcac aacaaaacta actaatacta acatctcaga cgctcaggaa 180

atagaaaccg tctgaactat cctgcccgcc atcatcctag tcctcatcgc cctcccatcc 240

ctacgcatcc tttacataac agacgaggtc aacgatccct cccttaccat caaatcaatt 300

ggccaccaat ggtactgaac ctacgagtac accgactacg gcggactaat cttcaactcc 360

tacatacttc ccccattatt cctagaacca ggcgacctgc gactccttga cgttgacaat 420

cgagtagtac tcccgattga agcccccatt cgtataataa ttacatcaca agacgtcttg 480

cactcatgag ctgtccccac attaggctta aaaacagatg caattcccgg acgtctaaac 540

caaaccactt tcaccgctac acgaccgggg gtatactacg gtcaatgctc tgaaatctgt 600

ggagcaaacc acagtttcat gcccatcgtc ctagacctaa cctgactaga aaagctatta 660

cctaaaacaa tttcacagca ccaaatctcc acctccatca tcacctcaac ccaaaaaggc 720

ataattaaac tttacttcct ctctttcttc ttcccactca tcctaaccct actcctaatc 780

acataa 786

<210> 14
<211> 951
<212> DNA
<213> Artificial

<220>
<223> cDNA

<400> 14
atgaacgaaa atctgttcgc ttcattcatt gcccccacaa tcctaggcct acccgccgca 60

gtactgatca ttctatttcc ccctctattg atccccacct ccaaatatct catcaacaac 120

cgactaatca ccacccaaca atgactaatc aaactaacct caaaacaaat gataaccata 180

cacaacacta aaggacgaac ctgatctctt atactagtat ccttaatcat ttttattgcc 240

acaactaacc tcctcggact cctgcctcac tcatttacac caaccaccca actatctata 300

aacctagcca tggccatccc cttatgagcg ggcacagtga ttataggctt tcgctctaag 360

attaaaaatg ccctagccca cttcttacca caaggcacac ctacacccct tatccccata 420

ctagttatta tcgaaaccat cagcctactc attcaaccaa tagccctggc cgtacgccta 480

accgctaaca ttactgcagg ccacctactc atgcacctaa ttggaagcgc caccctagca 540

atatcaacca ttaaccttcc ctctacactt atcatcttca caattctaat tctactgact 600

atcctagaaa tcgctgtcac tttcctagga cttctaacag ccctagacct caactaccta 660

accaacaaac ttaaaataaa atccccacta tgcacatttt atttctccaa catactcgga 720

ttctacccta gcatcacaca ccgcacaatc ccctatctag gccttcttac gagccaaaac 780

ctgcccctac tcctcctaga cctaacctga ctagaaaagc tattacctaa aacaatttca 840

cagcaccaaa tctccacctc catcatcacc tcaacccaaa aaggcataat taaactttac 900

ttcctctctt tcttcttccc actcatccta accctactcc taatcacata a 951

<210>  15
<211>  1905
<212>  DNA
<213>  Artificial

<220>
<223>  cDNA

<400>  15
atgaacgaaa atctgttcgc ttcattcatt gcccccacaa tcctaggcct acccgccgca      60

gtactgatca ttctatttcc ccctctattg atccccacct ccaaatatct catcaacaac     120

cgactaatca ccacccaaca atgactaatc aaactaacct caaaacaaat gataaccata     180

cacaacacta aaggacgaac ctgatctctt atactagtat ccttaatcat ttttattgcc     240

acaactaacc tcctcggact cctgcctcac tcatttacac caaccaccca actatctata     300

aacctagcca tggccatccc cttatgagcg ggcacagtga ttataggctt tcgctctaag     360

attaaaaatg ccctagccca cttcttacca caaggcacac ctacacccct tatccccata     420

ctagttatta tcgaaaccat cagcctactc attcaaccaa tagccctggc cgtacgccta     480

accgctaaca ttactgcagg ccacctactc atgcacctaa ttggaagcgc caccctagca     540

atatcaacca ttaaccttcc ctctacactt atcatcttca caattctaat tctactgact     600

atcctagaaa tcgctgtcgc cttaatccaa gcctacgttt tcacacttct agtaagcctc     660

tacctacact ccaactcatg agacccacaa caaatagccc ttctaaacgc taatccaagc     720

ctcaccccac tactaggcct cctcctagca gcagcaggca aatcagccca attaggtctc     780

cacccctgac tcccctcagc catagaaggc cccaccccag tctcagccct actccactca     840

agcactatag ttgtagcagg aatcttctta ctcatccgct tccacccccc agcagaaaat     900

agcccactaa tccaaactct aacactatgc ttaggcgcta tcaccactct gttcgcagca     960

gtctgcgccc ttacacaaaa tgacatcaaa aaaatcgtag ccttctccac ttcaagtcaa    1020

ctaggactca taatagttac aatcggcatc aaccaaccac acctagcatt cctgcacatc    1080

tgtacccacg ccttcttcaa agccatacta tttatgtgct ccgggtccat catccacaac    1140

cttaacaatg aacaagatat tcgaaaaata ggaggactac tcaaaaccat acctctcact    1200

tcaacctccc tcaccattgg cagcctagca ttagcaggaa tacctttcct cacaggtttc    1260

tactccaaag accacatcat cgaaaccgca aacatatcat acacaaacgc ctgagcccta    1320

tctattactc tcatcgctac ctccctgaca agcgcctata gcactcgaat aattcttctc    1380

accctaacag gtcaacctcg cttccccacc cttactaaca ttaacgaaaa taaccccacc    1440

ctactaaacc ccattaaacg cctggcagcc ggaagcctat tcgcaggatt tctcattact    1500

aacaacattt cccccgcatc ccccttccaa acaacaatcc ccctctacct aaaactcaca    1560

gccctcgctg tcactttcct aggacttcta acagccctag acctcaacta cctaaccaac    1620

aaacttaaaa taaaatcccc actatgcaca ttttatttct ccaacatact cggattctac 1680

cctagcatca cacaccgcac aatcccctat ctaggccttc ttacgagcca aaacctgccc 1740

ctactcctcc tagacctaac ctgactagaa aagctattac ctaaaacaat ttcacagcac 1800

caaatctcca cctccatcat cacctcaacc caaaaaggca taattaaact ttacttcctc 1860

tctttcttct tcccactcat cctaacccta ctcctaatca cataa 1905

<210> 16
<211> 1545
<212> DNA
<213> Artificial

<220>
<223> cDNA

<400> 16
   atgacccacc aatcacatgc ctatcatata gtaaaaccca gcccatgacc cctaacaggg 60
   gccctctcag ccctcctaat gacctccggc ctagccatgt gatttcactt ccactccata 120
   acgctcctca tactaggcct actaaccaac acactaacca tataccaatg atggcgcgat 180
   gtaacacgag aaagcacata ccaaggccac cacacaccac ctgtccaaaa aggccttcga 240
   tacgggataa tcctatttat tacctcagaa gtttttttct tcgcaggatt tttctgagcc 300
   ttttaccact ccagcctagc ccctaccccc caattaggag ggcactggcc cccaacaggc 360
   atcaccccac tactaggcct cctcctagca gcagcaggca aatcagccca attaggtctc 420
   cacccctgac tcccctcagc catagaaggc cccaccccag tctcagccct actccactca 480
   agcactatag ttgtagcagg aatcttctta ctcatccgct tccaccccct agcagaaaat 540
   agcccactaa tccaaactct aacactatgc ttaggcgcta tcaccactct gttcgcagca 600
   gtctgcgccc ttacacaaaa tgacatcaaa aaaatcgtag ccttctccac ttcaagtcaa 660
   ctaggactca taatagttac aatcggcatc aaccaaccac acctagcatt cctgcacatc 720
   tgtacccacg ccttcttcaa agccatacta tttatgtgct ccgggtccat catccacaac 780
   cttaacaatg aacaagatat tcgaaaaata ggaggactac tcaaaaccat acctctcact 840
   tcaacctccc tcaccattgg cagcctagca ttagcaggaa tacctttcct cacaggtttc 900
   tactccaaag accacatcat cgaaaccgca aacatatcat acacaaacgc ctgagcccta 960
   tctattactc tcatcgctac ctccctgaca agcgcctata gcactcgaat aattcttctc 1020
   accctaacag gtcaacctcg cttccccacc cttactaaca ttaacgaaaa taaccccacc 1080
   ctactaaacc ccattaaacg cctggcagcc ggaagcctat tcgcaggatt tctcattact 1140
   aacaacattt cccccgcatc ccccttccaa acaacaatcc ccctctacct aaaactcaca 1200
   gccctcgctg tcactttcct aggacttcta acagccctag acctcaacta cctaaccaac 1260
   aaacttaaaa taaaatcccc actatgcaca ttttatttct ccaacatact cggattctac 1320
   cctagcatca cacaccgcac aatcccctat ctaggccttc ttacgagcca aaacctgccc 1380
   ctactcctcc tagacctaac ctgactagaa aagctattac ctaaaacaat ttcacagcac 1440
   caaatctcca cctccatcat cacctcaacc caaaaaggca taattaaact ttacttcctc 1500
   tctttcttct tcccactcat cctaacccta ctcctaatca cataa 1545

<210> 17
<211> 1629
<212> DNA
<213> Artificial

<220>
<223> cDNA

<400> 17
ataaacttcg ccttaatttt aataatcaac accctcctag ccttactact aataattatt 60

acattttgac taccacaact caacggctac atagaaaaat ccacccctta cgagtgcggc 120

ttcgacccta tatcccccgc ccgcgtccct ttctccataa aattcttctt agtagctatt 180

accttcttat tatttgatct agaaattgcc ctccttttac ccctaccatg agccctacaa 240

acaactaacc tgccactaat agttatgtca tccctcttat taatcatcat cctagcccta 300

agtctggcca acacagcagc cattcaagca atcctataca accgtatcgg cgatatcggt 360

ttcatcctcg ccttagcatg atttatccta cactccaact catgagaccc acaacaaata 420

gcccttctaa acgctaatcc aagcctcacc ccactactag gcctcctcct agcagcagca 480

ggcaaatcag cccaattagg tctccacccc tgactcccct cagccataga aggccccacc 540

ccagtctcag ccctactcca ctcaagcact atagttgtag caggaatctt cttactcatc 600

cgcttccacc ccctagcaga aaatagccca ctaatccaaa ctctaacact atgcttaggc 660

gctatcacca ctctgttcgc agcagtctgc gcccttacac aaaatgacat caaaaaaatc 720

gtagccttct ccacttcaag tcaactagga ctcataatag ttacaatcgg catcaaccaa 780

ccacacctag cattcctgca catctgtacc cacgccttct tcaaagccat actatttatg 840

tgctccgggt ccatcatcca caaccttaac aatgaacaag atattcgaaa aataggagga 900

ctactcaaaa ccatacctct cacttcaacc tccctcacca ttggcagcct agcattagca 960

ggaatacctt tcctcacagg tttctactcc aaagaccaca tcatcgaaac cgcaaacata 1020

tcatacacaa acgcctgagc cctatctatt actctcatcg ctacctccct gacaagcgcc 1080

tatagcactc gaataattct tctcacccta acaggtcaac ctcgcttccc cacccttact 1140

aacattaacg aaaataaccc caccctacta aaccccatta aacgcctggc agccggaagc 1200

ctattcgcag gatttctcat tactaacaac atttcccccg catccccctt ccaaacaaca 1260

atccccctct acctaaaact cacagccctc gctgtcactt tcctaggact tctaacagcc 1320

ctagacctca actacctaac caacaaactt aaaataaaat ccccactatg cacattttat 1380

ttctccaaca tactcggatt ctaccctagc atcacacacc gcacaatccc ctatctaggc 1440

cttcttacga gccaaaacct gcccctactc ctcctagacc taacctgact agaaaagcta 1500

ttacctaaaa caatttcaca gcaccaaatc tccacctcca tcatcacctc aacccaaaaa 1560

ggcataatta aactttactt cctctctttc ttcttcccac tcatcctaac cctactccta 1620

atcacataa 1629

<210> 18
<211> 642
<212> DNA
<213> Artificial

<220>
<223> cDNA

<400> 18
atgctaaaac taatcgtccc aacaattata ttactaccac tgacatgact ttccaaaaaa 60

cacataattt gaatcaacac aaccacccac agcctaatta ttagcatcat ccctctacta 120

ttttttaacc aaatcaacaa caacctattt agctgttccc caaccttttc ctccgacccc 180

ctaacaaccc ccctcctaat actaactacc tgactcctac ccctcacaat catggcaagc 240

caacgccact tatccagtga accactatca cgaaaaaaac tctacctctc tatactaatc 300

tccctacaaa tctccttaat tataacattc acagccacag aactaatcat attttatatc 360

ttcttcgaaa ccacacttat ccccaccttg gctatcatca cccgatgagg caaccagcca 420

gaacgcctga acgcaggcac atacttccta ttctacaccc tagtaggctc cctgccccta 480

ctcctcctag acctaacctg actagaaaag ctattaccta aaacaatttc acagcaccaa 540

atctccacct ccatcatcac ctcaacccaa aaaggcataa ttaaacttta cttcctctct 600

ttcttcttcc cactcatcct aaccctactc ctaatcacat aa 642

<210> 19
<211> 129
<212> DNA
<213> Artificial

<220>
<223> cDNA

<400> 19
atgccccaac taaatactac cgtatggccc accataatta cccccatact ccttacacta 60

ttcctcatca cccaactaaa aatattaaac acaaactacc acctacctcc ctcaccattg 120

gcagcctag 129

<210> 20
<211> 1147

<212>  DNA
<213>  Artificial

<220>
<223>  cDNA

<400>  20
atactactaa tctcatcaat acaacccccg cccatcctac ccagcacaca cacaccgctg      60

ctaaccccat accccgaaaa tgttggttat acccttcccg tactaattaa tcccctggcc     120

caacccgtca tctactctac catctttgca ggcacactca tcacagcgct aagctcgcac     180

tgatttttta cctgagtagg cctagaaata aacatgctag cttttattcc agttctaacc     240

aaaaaaataa accctcgttc cacagaagct gccatcaagt atttcctcac gcaagcaacc     300

gcatccataa tccttctaat agctatcctc ttcaacaata tactctccgg acaatgaacc     360

ataaccaata ctaccaatca atactcatca ttaataatca taatagctat agcaataaaa     420

ctaggaatag ccccctttca cttctgagtc ccagaggtta cccaaggcac ccctctgaca     480

tccggcctgc ttcttctcac atgacaaaaa ctagccccca tctcaatcat ataccaaatc     540

tctccctcac taaacgtaag ccttctcctc actctctcaa tcttatccat catagcaggc     600

agttgaggtg gattaaacca aacccagcta cgcaaaatct tagcatactc ctcaattacc     660

cacataggat gaataatagc agttctaccg tacaacccta acataaccat tcttaattta     720

actatttata ttatcctaac tactaccgca ttcctactac tcaacttaaa ctccagcacc     780

acgaccctac tactatctcg cacctgaaac aagctaacat gactaacacc cttaattcca     840

tccaccctcc tctccctagg aggcctgccc ccgctaaccg gctttttgcc caaatgggcc     900

attatcgaag aattcacaaa aaacaatagc ctcatcatcc ccaccatcat agccaccatc     960

accctcctta acctctactt ctacctacgc ctaatctact ccacctcaat cacactactc    1020

cccatatcta acaacgtaaa aataaaatga cagtttgaac atacaaaacc caccccattc    1080

ctccccacac tcatcgccct taccacgcta ctcctaccta tctccccttt tatactaata    1140

atcttat                                                              1147


<210>  21
<211>  783
<212>  RNA
<213>  Human

<400>  21
auggcccacc auaauuaccc ccauacuccu uacacuauuc cucaucaccc aacuaaaaau      60

auuaaacaca aacuaccacc uaccucccuc accauuggca gccuagcauu agcaggaaua     120

ccuuuccuca cagguuucua cuccaaagac cacaucaucg aaaccgcaaa cauaucauac     180

acaaacgccu gagcccuauc uauuacucuc aucgcuaccu cccugacaag cgccuauagc     240

acucgaauaa uucuucucac ccuaacaggu caaccucgcu uccccacccu uacuaacauu     300

aacgaaaaua accccacccu acuaaacccc auuaaacgcc uggcagccgg aagccuauuc 360

gcaggauuuc ucauuacuaa caacauuucc cccgcauccc ccuuccaaac aacaaucccc 420

cucuaccuaa aacucacagc ccucgcuguc acuuuccuag gacuucuaac agcccuagac 480

cucaacuacc uaaccaacaa acuuaaaaua aaauccccac uaugcacauu uuauuucucc 540

aacauacucg gauucuaccc uagcaucaca caccgcacaa uccccuaucu aggccuucuu 600

acgagccaaa accugccccu acuccuccua gaccuaaccu gacuagaaaa gcuauuaccu 660

aaaacaauuu cacagcacca aaucuccacc uccaucauca ccucaaccca aaaaggcaua 720

auuaaacuuu acuuccucuc uuucuucuuc ccacucaucc uaacccuacu ccuaaucaca 780

uaa 783

<210> 22
<211> 300
<212> RNA
<213> Human

<400> 22
augccccuca uuuacauaaa uauuauacua gcauuuacca ucucacuucu aggaauacua 60

guauaucgcu cacaccucau auccucccua cuaugccuag aaggaauaau acuaucgcug 120

uucauuauag cuacucucau aacccucaac acccacuccc ucuuagccaa uauugugccu 180

auugccauac uagucuuugc cgccugcgaa gcagcggugg gccuagcccu acuagucuca 240

aucuccaaca cauauggccu agacuacgua cauaaccuaa cccuacuccu aaucacauaa 300

<210> 23
<211> 781
<212> RNA
<213> Human

<400> 23
auggcacaug cagcgcaagu aggucuacaa gacgcuacuu ccccuaucau agaagagcuu 60

aucaccuuuc augaucacgc ccucauaauc auuuuccuua ucugcuuccu aguccuguau 120

gcccuuuucc uaacacucac aacaaaacua acuaauacua acaucucaga cgcucaggaa 180

auagaaaccg ucugaacuau ccugcccgcc aucauccuag uccucaucgc ccucccaucc 240

cuacgcaucc uuuacauaac agacgagguc aacgaucccu cccuuaccau caaaucaauu 300

ggccaccaau gguacugaac cuacgaguac accgacuacg gcggacuaau cuucaacucc 360

uacauacuuc ccccauuauu ccuagaacca ggcgacccag acaauuauac ccuagccaac 420

cccuuaaaca ccccucccca caucaagccc gaaugauauu uccuauucgc cuacacaauu 480

cuccgauccg ucccuaacaa acuaggaggc guccuugccc uauuacuauc cauccucauc 540

cuagcaauaa uccccauccu ccauauaucc aaacaacaaa gcauaauauu ucgcccacua 600

agccaaucac uuuauugacu ccuagccgca gaccuccuca uucuaaccug aaucggagga 660

caaccaguaa gcuacccuuu uaccaucauu ggacaaguag cauccguacu auacuucaca 720

acaauccuaa uccuaauacc aacuaucucc cuaauugaaa acaaaauacu caaauggccc 780

u 781

<210> 24
<211> 565
<212> RNA
<213> Human

<400> 24
auggcacaug cagcgcaagu aggucuacaa gacgcuacuu ccccuaucau agaagagcuu 60

aucaccuuuc augaucacgc ccucauaauc auuuuccuua ucugcuuccu aguccuguau 120

gcccuuuucc uaacacucac aacaaaacua acuaauacua acaucucaga cgcucaggaa 180

auagaaaccg ucugaacuau ccugcccgcc aucauccuag uccucaucgc ccucccaucc 240

cuacgcaucc uuuacauaac agacgagguc aacgaucccu cccuuaccau caaaucaauu 300

ggccaccaau gguacugaac cuacgaguac accgacuacg gcggacuaau cuucaacucc 360

uacauacuuc ccccauuauu ccuagaacca ggcgaccugc gacuccuagc cgcagaccuc 420

cucauucuaa ccugaaucgg aggacaacca guaagcuacc cuuuuaccau cauuggacaa 480

guagcauccg uacuauacuu cacaacaauc cuaauccuaa uaccaacuau cucccuaauu 540

gaaaacaaaa uacucaaaug ggccu 565

<210> 25
<211> 1174
<212> RNA
<213> Human

<400> 25
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg ucugaacuau ccugcccgcc aucauccuag uccucaucgc ccucccaucc 240
   cuacgcaucc uuuacauaac agacgagguc aacgaucccu cccuuaccau caaaucaauu 300
   ggccaccaau gguacugaac cuacgaguac accgacuacg gcggacuaau cuucaacucc 360
   uacauacuuc ccccauuauu ccuagaacca ggcgaccugc gacuccuuga cguugacaau 420
   cgaguaguac ucccgauuga agcccccauu cguauaauaa uuacaucaca agacgucuug 480
   cacucaugag cuguccccac auuaggcuua aaaacagaug caauucccgg acgucuaaac 540
   caaaccacuu ucaccgcuac acgaccgggg guauacuacg gucaaugcuc ugaaaucugu 600
   ggagcaaacc acaguuucau gcccauauuc uugcacgaaa cgggaucaaa caacccccua 660
   ggaaucaccu cccauuccga uaaaaucacc uuccacccuu acuacacaau caaagacgcc 720
   cucggcuuac uucucuuccu ucuucccuua augacauuaa cacuauucuc accagaccuc 780
   cuaggcgacc cagacaauua uacccuagcc aaccccuuaa acaccccucc ccacaucaag 840
   cccgaaugau auuuccuauu cgccuacaca auucuccgau ccgucccuaa caaacuagga 900
   ggcguccuug cccuauuacu auccauccuc auccuagcaa uaauccccau ccuccauaua 960
   uccaaacaac aaagcauaau auuucgccca cuaagccaau cacuuuauug acuccuagcc 1020
   gcagaccucc ucauucuaac cugaaucgga ggacaaccag uaagcuaccc uuuuaccauc 1080
   auuggacaag uagcauccgu acuauacuuc acaacaaucc uaauccuaau accaacuauc 1140
   ucccuaauug aaaacaaaau acucaaaugg gccu 1174

<210> 26
<211> 1294
<212> RNA
<213> Human

<400> 26
augaacgaaa aucuguucgc uucauucauu gcccccacaa uccuaggccu acccgccgca 60

guacugauca uucuauuucc cccucuauug auccccaccu ccaaauaucu caucaacaac 120

cgacuaauca ccacccaaca augacuaauc aaacuaaccu caaaacaaau gauaaccaua 180

cacaacacua aaggacgaac cugaucucuu auacuaguau ccuuaaucau uuuuauugcc 240

acaacuaacc uccucggacu ccugccucac ucauuuacac caaccaccca acuaucuaua 300

aaccuagcca ugcacuacuc accagacgcc ucaaccgccu uuucaucaau cgcccacauc 360

acucgagacg uaaauuaugg cugaaucauc cgcuaccuuc acgccaaugg cgccucaaua 420

uucuuuaucu gccucuuccu acacaucggg cgaggccuau auuacggauc auuucucuac 480

ucagaaaccu gaaacaucgg cauuauccuc cugcuugcaa cuauagcaac agccuucaua 540

ggcuaugucc ucccgugagg ccaaauauca uucugagggg ccacaguaau uacaaacuua 600

cuauccgcca ucccauacau ugggacagac cuaguucaau gaaucugagg aggcuacuca 660

guagacaguc ccacccucac acgauucuuu accuuucacu ucaucuugcc cuucauuauu 720

gcagcccuag caacacucca ccuccuauuc uugcacgaaa cgggaucaaa caacccccua 780

ggaaucaccu cccauuccga uaaaaucacc uuccacccuu acuacacaau caaagacgcc 840

cucggcuuac uucucuuccu ucuucccuua augacauuaa cacuauucuc accagaccuc 900

cuaggcgacc cagacaauua uacccuagcc aaccccuuaa acaccccucc ccacaucaag 960

cccgaaugau auuuccuauu cgccuacaca auucuccgau ccgucccuaa caaacuagga 1020

ggcguccuug cccuauuacu auccauccuc auccuagcaa uaauccccau ccuccauaua 1080

uccaaacaac aaagcauaau auuucgccca cuaagccaau cacuuuauug acuccuagcc 1140

gcagaccucc ucauucuaac cugaaucgga ggacaaccag uaagcuaccc uuuuaccauc 1200

auuggacaag uagcauccgu acuauacuuc acaacaaucc uaauccuaau accaacuauc 1260

ucccuaauug aaaacaaaau acucaaaugg gccu 1294

<210> 27
<211> 1228
<212> RNA
<213> Human

<400> 27
augccccuca uuuacauaaa uauuauacua gcauuuacca ucucacuucu aggaauacua 60

guauaucgcu cacaccucau auccucccua cuaugccuag aaggaauaau acuaucgcug 120

uucauuauag cuacucucau aacccucaac acccacuccc ucuuagccaa uauugugccu 180

auugccauac uagucuuugg cgccugccug auccuccaaa ucaccacagg acuauuccua 240

gccaugcacu acucaccaga cgccucaacc gccuuuucau caaucgccca caucacucga 300

gacguaaauu auggcugaau cauccgcuac cuucacgcca auggcgccuc aauauucuuu 360

aucugccucu uccuacacau cgggcgaggc cuauauuacg gaucauuucu cuacucagaa 420

accugaaaca ucggcauuau ccuccugcuu gcaacuauag caacagccuu cauaggcuau 480

guccucccgu gaggccaaau aucauucuga ggggccacag uaauuacaaa cuuacuaucc 540

gccaucccau acauugggac agaccuaguu caaugaaucu gaggaggcua cucaguagac 600

agucccaccc ucacacgauu cuuuaccuuu cacuucaucu ugcccuucau uauugcagcc 660

cuagcaacac uccaccuccu auucuugcac gaaacgggau caaacaaccc ccuaggaauc 720

accucccauu ccgauaaaau caccuuccac ccuuacuaca caaucaaaga cgcccucggc 780

uuacuucucu uccuucucuc cuuaaugaca uuaacacuau ucucaccaga ccuccuaggc 840

gacccagaca auuauacccu agccaacccc uuaaacaccc cuccccacau caagcccgaa 900

ugauauuucc uauucgccua cacaauucuc cgauccgucc cuaacaaacu aggaggcguc 960

cuugcccuau uacuauccau ccucauccua gcaauaaucc ccauccucca uauauccaaa 1020

caacaaagca uaauauuucg cccacuaagc caaucacuuu auugacuccu agccgcagac 1080

cuccucauuc uaaccugaau cggaggacaa ccaguaagcu acccuuuuac caucauugga 1140

caaguagcau ccguacuaua cuucacaaca auccuaaucc uaauaccaac uaucucccua 1200

auugaaaaca aaauacucaa augggccu 1228

<210> 28
<211> 522
<212> RNA
<213> Human

<400> 28

auguucgccg accguugacu auucucuaca aaccacaaag acauuggaac acuauaccua 60

uuauucggcg caugagcugg aguccuaggc acagcucuaa gccuccuuau ucgagccgag 120

cugggccagc caggcaaccu ucuagguaac gaccacaucu acaacguuau cgucacagcc 180

cucgcuguca cuuuccuagg acuucuaaca gcccuagacc ucaacuaccu aaccaacaaa 240

cuuaaaauaa aauccccacu augcacauuu uauuucucca acauacucgg auucuacccu 300

agcaucacac accgcacaau ccccuaucua ggccuucuua cgagccaaaa ccugccccua 360

cuccuccuag accuaaccug acuagaaaag cuauuaccua aaacaauuuc acagcaccaa 420

aucuccaccu ccaucaucac cucaacccaa aaaggcauaa uuaaacuuua cuuccucucu 480

uucuucuucc cacucauccu aacccuacuc cuaaucacau aa 522

<210> 29
<211> 582
<212> RNA
<213> Human

<400> 29
auguucgccg accguugacu auucucuaca aaccacaaag acauuggaac acuauaccua 60

uuauucggcg caugagcugg aguccuaggc acagcucuaa gccuccuuau ucgagccgag 120

cugggccagc caggcaaccu ucuagguaac gaccacaucu acaacguuau cgucacagcc 180

caugcauuug uaauaaucuu cuucauagua auacccauca uaaucggagg cuuuggcaac 240

ugacuaguuc cccuaauaau cggugccccc gauauggcgu uuccccgcau aaacaacaua 300

agcuucugac ucuuaccucc cucucuccua cuccugcucg caucugcuau aguggaggcc 360

ggagcaggaa cagguugaac agucuacccu cccuuagcag ggaacuacuc ccacccugga 420

gcccuccuag accuaaccug acuagaaaag cuauuaccua aaacaauuuc acagcaccaa 480

aucuccaccu ccaucaucac cucaacccaa aaaggcauaa uuaaacuuua cuuccucucu 540

uucuucuucc cacucauccu aacccuacuc cuaaucacau aa 582

<210> 30
<211> 2208
<212> RNA
<213> Human

<400> 30
auguucgccg accguugacu auucucuaca aaccacaaag acauuggaac acuauaccua 60

uuauucggcg caugagcugg aguccuaggc acagcucuaa gccuccuuau ucgagccgag 120

cugggccagc caggcaaccu ucuagguaac gaccacaucu acaacguuau cgucacagcc 180

caugcauuug uaauaaucuu cuucauagua auacccauca uaaucggagg cuuuggcaac 240

ugacuaguuc cccuaauaau cggugccccc gauauggcgu uuccccgcau aaacaacaua 300

agcuucugac ucuuaccucc cucucuccua cuccugcucg caucugcuau aguggaggcc 360

ggagcaggaa cagguugaac agucuacccu cccuuagcag ggaacuacuc ccacccugga 420
gccuccguag accuaaccau cuucuccuua caccuagcag gugucuccuc uaucuuaggg 480
gccaucaauu ucaucacaac aauuaucaau auaaaacccc cugccauaac ccaauaccaa 540
acgccccucu ucgucugauc cguccuaauc acagcagucc uacuucuccu aucucuccca 600
guccuagcug cuggcaucac uauacuacua acagaccgca accucaacac caccuucuuc 660
gaccccgccg gaggaggaga ccccauucua uaccaacacc uauucugauu uuucggucac 720
ccugaaguuu auauucuuau ccuaccaggc uucggaauaa ucucccauau uguaacuuac 780
uacuccggaa aaaaagaacc auuuggauac auagguaugg ucugagcuau gauaucaauu 840
ggcuuccuag gguuuaucgu gugagcacac cauauauuua caguaggaau agacguagac 900
acacgagcau auuucaccuc cgcuaccaua aucaucgcua uccccaccgg cgucaaagua 960
uuuagcugac ucgccacacu ccacggaagc aauaugaaau gaucugcugc agugcucuga 1020
gcccuaggau ucaucuuucu uuucaccgua gguggccuga cuggcauugu auuagcaaac 1080
ucaucacuag acaucguacu acacgacacg uacuacguug uagcccacuu ccacuauguc 1140
cuaucaauag gagcuguauu ugccaucaua ggaggcuuca uucacugauu uccccuauuc 1200
ucaggcuaca cccuagacca aaccuacgcc aaaauccauu ucacuaucau auucaucggc 1260
guaaaucuaa cuuucuuccc acaacacuuu cucggccuau ccggaaugcc ccgacguuac 1320
ucggacuacc ccgaugcaua caccacauga aacauccuau caucuguagg cucauucauu 1380
ucucuaacag caguaauauu aauaauuuuc augauuugag aagccuucgc uucgaagcga 1440
aaaguccuaa uaguagaaga acccuccaua aaccuggagu gacuauaugg augcccccca 1500
cccuaccaca cauucgaaga acccguauac auaaaagcag gaauaccuuu ccucacaggu 1560
uucuacucca aagaccacau caucgaaacc gcaaacauau cauacacaaa cgccugagcc 1620
cuaucuauua cucucaucgc uaccucccug acaagcgccu auagcacucg aauaauucuu 1680
cucacccuaa caggucaacc ucgcuucccc acccuuacua acauuaacga aaauaacccc 1740
acccuacuaa accccauuaa acgccuggca gccggaagcc uauucgcagg auuucucauu 1800
acuaacaaca uuucccccgc aucccccuuc caaacaacaa ucccccucua ccuaaaacuc 1860
acagcccucg cugucacuuu ccuaggacuu cuaacagccc uagaccucaa cuaccuaacc 1920
aacaaacuua aaauaaaauc cccacuaugc acauuuuauu ucuccaacau acucggauuc 1980
uacccuagca ucacacaccg cacaaucccc uaucuaggcc uucuuacgag ccaaaaccug 2040
ccccuacucc uccuagaccu aaccugacua gaaaagcuau uaccuaaaac aauuucacag 2100
caccaaaucu ccaccuccau caucaccuca acccaaaaag gcauaauuaa acuuuacuuc 2160
cucucuuucu ucuucccacu cauccuaacc cuacuccuaa ucacauaa 2208

<210> 31
<211> 807
<212> RNA
<213> Human

<400> 31
   auggcacaug cagcgcaagu aggucuacaa gacgcuacuu ccccuaucau agaagagcuu 60
   aucaccuuuc augaucacgc ccucauaauc auuuuccuua ucugcuuccu aguccuguau 120
   gcccuuuucc uaacacucac aacaaaacua acuaauacua acaucucaga cgcucaggaa 180
   auagaaaccg caaacauauc auacacaaac gccugagccc uaucuauuac ucucaucgcu 240
   accucccuga caagcgccua uagcacucga auaauucuuc ucacccuaac aggucaaccu 300
   cgcuucccca cccuuacuaa cauuaacgaa aauaacccca cccuacuaaa ccccauuaaa 360
   cgccuggcag ccggaagccu auucgcagga uuucucauua cuaacaacau uucccccgca 420
   ucccccuucc aaacaacaau cccccucuac cuaaaacuca cagcccucgc ugucacuuuc 480
   cuaggacuuc uaacagcccu agaccucaac uaccuaacca acaaacuuaa aauaaaaucc 540
   ccacuaugca cauuuuauuu cuccaacaua cucggauucu acccuagcau cacacaccgc 600
   acaaucccca aucuaggccu ucuuacgagc caaaaccugc cccuacuccu ccuagaccua 660
   accugacuag aaaagcuauu accuaaaaca auuucacagc accaaaucuc caccuccauc 720
   aucaccucaa cccaaaaagg cauaauuaaa cuuuacuucc ucucuuucuu cuucccacuc 780
   auccuaaccc uacuccuaau cacauaa 807

<210> 32
<211> 786
<212> RNA
<213> Human

<400> 32
auggcacaug cagcgcaagu aggucuacaa gacgcuacuu ccccuaucau agaagagcuu 60

aucaccuuuc augaucacgc ccucauaauc auuuuccuua ucugcuuccu aguccuguau 120

gcccuuuucc uaacacucac aacaaaacua acuaauacua acaucucaga cgcucaggaa 180

auagaaaccg ucugaacuau ccugcccgcc aucauccuag uccucaucgc ccucccaucc 240

cuacgcaucc uuuacauaac agacgagguc aacgaucccu cccuuaccau caaaucaauu 300

ggccaccaau gguacugaac cuacgaguac accgacuacg gcggacuaau cuucaacucc 360

uacauacuuc ccccauuauu ccuagaacca ggcgaccugc gacuccuuga cguugacaau 420

cgaguaguac ucccgauuga agcccccauu cguauaauaa uuacaucaca agacgucuug 480

cacucaugag cuguccccac auuaggcuua aaaacagaug caauucccgg acgucuaaac 540

caaaccacuu ucaccgcuac acgaccgggg guauacuacg gucaaugcuc ugaaaucugu 600

ggagcaaacc acaguuucau gcccaucguc cuagaccuaa ccugacuaga aaagcuauua 660

ccuaaaacaa uuucacagca ccaaaucucc accuccauca ucaccucaac ccaaaaaggc 720

auaauuaaac uuuacuuccu cucuuucuuc uucccacuca uccuaacccu acuccuaauc 780

acauaa 786

<210> 33
<211> 951
<212> RNA
<213> Human

<400> 33
augaacgaaa aucuguucgc uucauucauu gcccccacaa uccuaggccu acccgccgca 60

guacugauca uucuauuucc cccucuauug auccccaccu ccaaauaucu caucaacaac 120

cgacuaauca ccacccaaca augacuaauc aaacuaaccu caaaacaaau gauaaccaua 180

cacaacacua aaggacgaac cugaucucuu auacuaguau ccuuaaucau uuuuauugcc 240

acaacuaacc uccucggacu ccugccucac ucauuuacac caaccaccca acuaucuaua 300

aaccuagcca uggccauccc cuuaugagcg ggcacaguga uuauaggcuu ucgcucuaag 360

auuaaaaaug cccuagccca cuucuuacca caaggcacac cuacaccccu uauccccaua 420

cuaguuauua ucgaaaccau cagccuacuc auucaaccaa uagcccuggc cguacgccua 480

accgcuaaca uuacugcagg ccaccuacuc augcaccuaa uuggaagcgc cacccuagca 540

auaucaacca uuaaccuucc cucuacacuu aucaucuuca caauucuaau ucuacugacu 600

auccuagaaa ucgcugucac uuuccuagga cuucuaacag cccuagaccu caacuaccua 660

accaacaaac uuaaaauaaa auccccacua ugcacauuuu auuucuccaa cauacucgga 720

uucuacccua gcaucacaca ccgcacaauc cccuaucuag gccuucuuac gagccaaaac 780

cugccccuac uccuccuaga ccuaaccuga cuagaaaagc uauuaccuaa aacaauuuca 840

cagcaccaaa ucuccaccuc caucaucacc ucaacccaaa aaggcauaau uaaacuuuac 900

uuccucucuu ucuucuuccc acucauccua acccuacucc uaaucacaua a 951

<210> 34
<211> 1905
<212> RNA
<213> Human

<400> 34
augaacgaaa aucuguucgc uucauucauu gcccccacaa uccuaggccu acccgccgca 60

guacugauca uucuauuucc cccucuauug auccccaccu ccaaauaucu caucaacaac 120

cgacuaauca ccacccaaca augacuaauc aaacuaaccu caaaacaaau gauaaccaua 180

cacaacacua aaggacgaac cugaucucuu auacuaguau ccuuaaucau uuuuauugcc 240

acaacuaacc uccucggacu ccugccucac ucauuuacac caaccaccca acuaucuaua 300

aaccuagcca uggccauccc cuuaugagcg ggcacaguga uuauaggcuu ucgcucuaag 360

auuaaaaaug cccuagccca cuucuuacca caaggcacac cuacaccccu uauccccaua 420

cuaguuauua ucgaaaccau cagccuacuc auucaaccaa uagcccuggc cguacgccua 480

accgcuaaca uuacugcagg ccaccuacuc augcaccuaa uuggaagcgc cacccuagca 540

auaucaacca uuaaccuucc cucuacacuu aucaucuuca caauucuaau ucuacugacu 600

auccuagaaa ucgcugucgc cuuaauccaa gccuacguuu ucacacuucu aguaagccuc 660

uaccuacacu ccaacucaug agacccacaa caaauagccc uucuaaacgc uaauccaagc 720

cucaccccac uacuaggccu ccuccuagca gcagcaggca aaucagccca auuaggucuc 780

caccccugac uccccucagc cauagaaggc cccaccccag ucucagcccu acuccacuca 840

agcacuauag uuguagcagg aaucuucuua cucauccgcu uccacccccu agcagaaaau 900

agcccacuaa uccaaacucu aacacuaugc uuaggcgcua ucaccacucu guucgcagca 960

gucugcgccc uuacacaaaa ugacaucaaa aaaaucguag ccuucuccac uucaagucaa 1020

cuaggacuca uaauaguuac aaucggcauc aaccaaccac accuagcauu ccugcacauc 1080

uguacccacg ccuucuucaa agccauacua uuuaugugcu ccggguccau cauccacaac 1140

cuuaacaaug aacaagauau ucgaaaaaua ggaggacuac ucaaaaccau accucucacu 1200

ucaaccuccc ucaccauugg cagccuagca uuagcaggaa uaccuuuccu cacagguuuc 1260

uacuccaaag accacaucau cgaaaccgca aacauaucau acacaaacgc cugagcccua 1320

ucuauuacuc ucaucgcuac cucccugaca agcgccuaua gcacucgaau aauucuucuc 1380

acccuaacag gucaaccucg cuuccccacc cuuacuaaca uuaacgaaaa uaaccccacc 1440

cuacuaaacc ccauuaaacg ccuggcagcc ggaagccuau ucgcaggauu ucucauuacu 1500

aacaacauuu cccccgcauc ccccuuccaa acaacaaucc cccucuaccu aaaacucaca 1560

gcccucgcug ucacuuuccu aggacuucua acagcccuag accucaacua ccuaaccaac 1620

aaacuuaaaa uaaaaucccc acuaugcaca uuuuauuucu ccaacauacu cggauucuac 1680

ccuagcauca cacaccgcac aauccccuau cuaggccuuc uuacgagcca aaaccugccc 1740

cuacuccucc uagaccuaac cugacuagaa aagcuauuac cuaaaacaau uucacagcac 1800

caaaucucca ccuccaucau caccucaacc caaaaaggca uaauuaaacu uuacuuccuc 1860

ucuuucuucu ucccacucau ccuaacccua cuccuaauca cauaa 1905

<210> 35
<211> 1545
<212> RNA
<213> Human

<400> 35
augacccacc aaucacaugc cuaucauaua guaaaaccca gcccaugacc ccuaacaggg 60

gcccucucag cccuccuaau gaccuccggc cuagccaugu gauuucacuu ccacuccaua 120

acgcuccuca uacuaggccu acuaaccaac acacuaacca uauaccaaug auggcgcgau 180

guaacacgag aaagcacaua ccaaggccac cacacaccac cuguccaaaa aggccuucga 240

uacgggauaa uccuauuuau uaccucagaa guuuuuuucu ucgcaggauu uuucugagcc 300

uuuuaccacu ccagccuagc cccuaccccc caauuaggag ggcacuggcc cccaacaggc 360

aucaccccac uacuaggccu ccuccuagca gcagcaggca aaucagccca auuaggucuc 420

caccccugac uccccucagc cauagaaggc cccaccccag ucucagcccu acuccacuca 480

agcacuauag uuguagcagg aaucuucuua cucauccgcu uccacccccu agcagaaaau 540

agcccacuaa uccaaacucu aacacuaugc uuaggcgcua ucaccacucu guucgcagca 600

gucugcgccc uuacacaaaa ugacaucaaa aaaaucguag ccuucuccac uucaagucaa 660

cuaggacuca uaauaguuac aaucggcauc aaccaaccac accuagcauu ccugcacauc 720

uguacccacg ccuucuucaa agccauacua uuuaugugcu ccggguccau cauccacaac 780

cuuaacaaug aacaagauau ucgaaaaaua ggaggacuac ucaaaaccau accucucacu 840

ucaaccuccc ucaccauugg cagccuagca uuagcaggaa uaccuuuccu cacagguuuc 900

uacuccaaag accacaucau cgaaaccgca aacauaucau acacaaacgc cugagcccua 960

ucuauuacuc ucaucgcuac cucccugaca agcgccuaua gcacucgaau aauucuucuc 1020

acccuaacag gucaaccucg cuuccccacc cuuacuaaca uuaacgaaaa uaaccccacc 1080

cuacuaaacc ccauuaaacg ccuggcagcc ggaagccuau ucgcaggauu ucucauuacu 1140

aacaacauuu cccccgcauc ccccuuccaa acaacaaucc cccucuaccu aaaacucaca 1200

gcccucgcug ucacuuuccu aggacuucua acagcccuag accucaacua ccuaaccaac 1260

aaacuuaaaa uaaaaucccc acuaugcaca uuuuauuucu ccaacauacu cggauucuac 1320

ccuagcauca cacaccgcac aauccccuau cuaggccuuc uuacgagcca aaaccugccc 1380

cuacuccucc uagaccuaac cugacuagaa aagcuauuac cuaaaacaau uucacagcac 1440

caaaucucca ccuccaucau caccucaacc caaaaaggca uaauuaaacu uuacuuccuc 1500

ucuuucuucu ucccacucau ccuaacccua cuccuaauca cauaa 1545

<210> 36
<211> 1629
<212> RNA
<213> Human

<400> 36
auaaacuucg ccuuaauuuu aauaaucaac acccuccuag ccuuacuacu aauaauuauu 60

acauuuugac uaccacaacu caacggcuac auagaaaaau ccaccccuua cgagugcggc 120

uucgacccua uaucccccgc ccgcgucccu uucuccauaa aauucuucuu aguagcuauu 180

accuucuuau uauuugaucu agaaauugcc cuccuuuuac cccuaccaug agcccuacaa 240

acaacuaacc ugccacuaau aguuauguca ucccucuuau uaaucaucau ccuagcccua 300

agucuggcca acacagcagc cauucaagca auccuauaca accguaucgg cgauaucggu 360

uucauccucg ccuuagcaug auuuauccua cacuccaacu caugagaccc acaacaaaua 420

gcccuucuaa acgcuaaucc aagccucacc ccacuacuag gccuccuccu agcagcagca 480

ggcaaaucag cccaauuagg ucuccacccc ugacuccccu cagccauaga aggccccacc 540

ccagucucag cccuacucca cucaagcacu auaguuguag caggaaucuu cuuacucauc 600

cgcuuccacc cccuagcaga aaauagccca cuaauccaaa cucuaacacu augcuuaggc 660

gcuaucacca cucuguucgc agcagucugc gcccuuacac aaaaugacau caaaaaaauc 720

guagccuucu ccacuucaag ucaacuagga cucauaauag uuacaaucgg caucaaccaa 780

ccacaccuag cauuccugca caucuguacc cacgccuucu ucaaagccau acuauuuaug 840

ugcuccgggu ccaucaucca caaccuuaac aaugaacaag auauucgaaa aauaggagga 900

cuacucaaaa ccauaccucu cacuucaacc ucccucacca uuggcagccu agcauuagca 960

ggaauaccuu uccucacagg uuucuacucc aaagaccaca ucaucgaaac cgcaaacaua 1020

ucauacacaa acgccugagc ccuaucuauu acucucaucg cuaccucccu gacaagcgcc 1080

uauagcacuc gaauaauucu ucucacccua acaggucaac cucgcuuccc cacccuuacu 1140

aacauuaacg aaaauaaccc cacccuacua aaccccauua aacgccuggc agccggaagc 1200

cuauucgcag gauuucucau uacuaacaac auuucccccg caucccccuu ccaaacaaca 1260

aucccccucu accuaaaacu cacagcccuc gcugucacuu uccuaggacu ucuaacagcc 1320

cuagaccuca acuaccuaac caacaaacuu aaaauaaaau ccccacuaug cacauuuuau 1380

uucuccaaca uacucggauu cuacccuagc aucacacacc gcacaauccc cuaucuaggc 1440

cuucuuacga gccaaaaccu gccccuacuc cuccuagacc uaaccugacu agaaaagcua 1500

uuaccuaaaa caauuucaca gcaccaaauc uccaccucca ucaucaccuc aacccaaaaa 1560

ggcauaauua aacuuuacuu ccucucuuuc uucuucccac ucauccuaac ccuacuccua 1620

aucacauaa 1629

<210> 37
<211> 642
<212> RNA
<213> Human

<400> 37
augcuaaaac uaaucguccc aacaauuaua uuacuaccac ugacaugacu uuccaaaaaa 60

cacauaauuu gaaucaacac aaccacccac agccuaauua uuagcaucau cccucuacua 120

uuuuuuaacc aaaucaacaa caaccuauuu agcuguuccc caaccuuuuc cuccgacccc 180

cuaacaaccc cccuccuaau acuaacuacc ugacuccuac cccucacaau cauggcaagc 240

caacgccacu uauccaguga accacuauca cgaaaaaaac ucuaccucuc uauacuaauc 300

ucccuacaaa ucuccuuaau uauaacauuc acagccacag aacuaaucau auuuuauauc 360

uucuucgaaa ccacacuuau ccccaccuug gcuaucauca cccgaugagg caaccagcca 420

gaacgccuga acgcaggcac auacuuccua uucuacaccc uaguaggcuc ccugccccua 480

cuccuccuag accuaaccug acuagaaaag cuauuaccua aaacaauuuc acagcaccaa 540

aucuccaccu ccaucaucac cucaacccaa aaaggcauaa uuaaacuuua cuuccucucu 600

uucuucuucc cacucauccu aacccuacuc cuaaucacau aa 642

<210> 38
<211> 129
<212> RNA
<213> Human

<400> 38
augccccaac uaaauacuac cguauggccc accauaauua cccccauacu ccuuacacua 60

uuccucauca cccaacuaaa aauauuaaac acaaacuacc accuaccucc cucaccauug 120

gcagccuag 129

<210> 39
<211> 1147
<212> RNA
<213> Human

<400> 39
auacuacuaa ucucaucaau acaacccccg cccauccuac ccagcacaca cacaccgcug 60

cuaaccccau accccgaaaa uguugguuau acccuucccg uacuaauuaa uccccuggcc 120

caacccguca ucuacucuac caucuuugca ggcacacuca ucacagcgcu aagcucgcac 180

ugauuuuuua ccugaguagg ccuagaaaua aacaugcuag cuuuuauucc aguucuaacc 240

aaaaaaauaa acccucguuc cacagaagcu gccaucaagu auuuccucac gcaagcaacc 300

gcauccauaa uccuucuaau agcuauccuc uucaacaaua uacucuccgg acaaugaacc 360

auaaccaaua cuaccaauca auacucauca uuaauaauca uaauagcuau agcaauaaaa 420

cuaggaauag ccccuuuuca cuucugaguc ccagagguua cccaaggcac cccucugaca 480

uccggccugc uucuucucac augacaaaaa cuagccccca ucucaaucau auaccaaauc 540

ucucccucac uaaacguaag ccuucuccuc acucucucaa ucuuauccau cauagcaggc 600

aguugaggug gauuaaacca aacccagcua cgcaaaaucu uagcauacuc cucaauuacc 660

cacauaggau gaauaauagc aguucuaccg uacaacccua acauaaccau ucuuaauuua 720

acuauuuaua uuauccuaac uacuaccgca uuccuacuac ucaacuuaaa cuccagcacc 780

acgacccuac uacuaucucg caccugaaac aagcuaacau gacuaacacc cuuaauucca 840

uccacccucc ucucccuagg aggccugccc ccgcuaaccg gcuuuuugcc caaaugggcc 900

auuaucgaag aauucacaaa aaacaauagc cucaucaucc ccaccaucau agccaccauc 960

acccuccuua accucuacuu cuaccuacgc cuaaucuacu ccaccucaau cacacuacuc 1020

cccauaucua acaacguaaa aauaaaauga caguuugaac auacaaaacc caccccauuc 1080

cuccccacac ucaucgcccu uaccacgcua cuccuaccua ucuccccuuu uauacuaaua 1140

aucuuau 1147

<210> 40
<211> 100
<212> PRT
<213> Artificial

<220>
<223> putative protein sequence

<220>
<221> misc_feature
<222> (100)..(100)
<223> Xaa can be any naturally occurring amino acid

<400> 40

Met Pro Leu Ile Tyr Met Asn Ile Met Leu Ala Phe Thr Ile Ser Leu
1               5                   10                  15

Leu Gly Met Leu Val Tyr Arg Ser His Leu Met Ser Ser Leu Leu Cys
            20                  25                  30

Leu Glu Gly Met Met Leu Ser Leu Phe Ile Met Ala Thr Leu Met Thr
        35                  40                  45

Leu Asn Thr His Ser Leu Leu Ala Asn Ile Val Pro Ile Ala Met Leu
    50                  55                  60

Val Phe Ala Ala Cys Glu Ala Ala Val Gly Leu Ala Leu Leu Val Ser
65                  70                  75                  80

Ile Ser Asn Thr Tyr Gly Leu Asp Tyr Val His Asn Leu Thr Leu Leu
                85                  90                  95

Leu Ile Thr Xaa
            100

<210> 41
<211> 261
<212> PRT
<213> Artificial

<220>
<223>  putative protein sequence


<220>
<221>  misc_feature
<222>  (261)..(261)
<223>  Xaa can be any naturally occurring amino acid

<400>  41

Met Ala His Ala Ala Gln Val Gly Leu Gln Asp Ala Thr Ser Pro Ile
1               5                   10                  15


Met Glu Glu Leu Ile Thr Phe His Asp His Ala Leu Met Ile Ile Phe
            20                  25                  30


Leu Ile Cys Phe Leu Val Leu Tyr Ala Leu Phe Leu Thr Leu Thr Thr
        35                  40                  45


Lys Leu Thr Asn Thr Asn Ile Ser Asp Ala Gln Glu Met Glu Thr Val
    50                  55                  60


Trp Thr Ile Leu Pro Ala Ile Ile Leu Val Leu Ile Ala Leu Pro Ser
65                  70                  75                  80


Leu Arg Ile Leu Tyr Met Thr Asp Glu Val Asn Asp Pro Ser Leu Thr
                85                  90                  95


Ile Lys Ser Ile Gly His Gln Trp Tyr Trp Thr Tyr Glu Tyr Thr Asp
            100                 105                 110


Tyr Gly Gly Leu Ile Phe Asn Ser Tyr Met Leu Pro Pro Leu Phe Leu
        115                 120                 125


Glu Pro Gly Asp Pro Asp Asn Tyr Thr Leu Ala Asn Pro Leu Asn Thr
    130                 135                 140


Pro Pro His Ile Lys Pro Glu Trp Tyr Phe Leu Phe Ala Tyr Thr Ile
145                 150                 155                 160


Leu Arg Ser Val Pro Asn Lys Leu Gly Gly Val Leu Ala Leu Leu Leu
                165                 170                 175


Ser Ile Leu Ile Leu Ala Met Ile Pro Ile Leu His Met Ser Lys Gln
            180                 185                 190


Gln Ser Met Met Phe Arg Pro Leu Ser Gln Ser Leu Tyr Trp Leu Leu
        195                 200                 205

Ala Ala Asp Leu Leu Ile Leu Thr Trp Ile Gly Gly Gln Pro Val Ser
    210                 215                 220

Tyr Pro Phe Thr Ile Ile Gly Gln Val Ala Ser Val Leu Tyr Phe Thr
225                 230                 235                 240

Thr Ile Leu Ile Leu Met Pro Thr Ile Ser Leu Ile Glu Asn Lys Met
                245                 250                 255

Leu Lys Trp Ala Xaa
            260

<210>  42
<211>  269
<212>  PRT
<213>  Artificial

<220>
<223>  putative protein sequence

<220>
<221>  misc_feature
<222>  (269)..(269)
<223>  Xaa can be any naturally occurring amino acid

<400>  42

Met Ala His Ala Ala Gln Val Gly Leu Gln Asp Ala Thr Ser Pro Ile
1               5                   10                  15

Met Glu Glu Leu Ile Thr Phe His Asp His Ala Leu Met Ile Ile Phe
            20                  25                  30

Leu Ile Cys Phe Leu Val Leu Tyr Ala Leu Phe Leu Thr Leu Thr Thr
        35                  40                  45

Lys Leu Thr Asn Thr Asn Ile Ser Asp Ala Gln Glu Met Glu Thr Ala
    50                  55                  60

Asn Met Ser Tyr Thr Asn Ala Trp Ala Leu Ser Ile Thr Leu Ile Ala
65                  70                  75                  80

Thr Ser Leu Thr Ser Ala Tyr Ser Thr Arg Met Ile Leu Leu Thr Leu
                85                  90                  95

Thr Gly Gln Pro Arg Phe Pro Thr Leu Thr Asn Ile Asn Glu Asn Asn
            100                 105                 110

Pro Thr Leu Leu Asn Pro Ile Lys Arg Leu Ala Ala Gly Ser Leu Phe
        115                 120                 125

Ala Gly Phe Leu Ile Thr Asn Asn Ile Ser Pro Ala Ser Pro Phe Gln
    130                 135                 140

Thr Thr Ile Pro Leu Tyr Leu Lys Leu Thr Ala Leu Ala Val Thr Phe
145                 150                 155                 160

Leu Gly Leu Leu Thr Ala Leu Asp Leu Asn Tyr Leu Thr Asn Lys Leu
                165                 170                 175

Lys Met Lys Ser Pro Leu Cys Thr Phe Tyr Phe Ser Asn Met Leu Gly
            180                 185                 190

Phe Tyr Pro Ser Ile Thr His Arg Thr Ile Pro Tyr Leu Gly Leu Leu
        195                 200                 205

Thr Ser Gln Asn Leu Pro Leu Leu Leu Leu Asp Leu Thr Trp Leu Glu
    210                 215                 220

Lys Leu Leu Pro Lys Thr Ile Ser Gln His Gln Ile Ser Thr Ser Ile
225                 230                 235                 240

Ile Thr Ser Thr Gln Lys Gly Met Ile Lys Leu Tyr Phe Leu Ser Phe
                245                 250                 255

Phe Phe Pro Leu Ile Leu Thr Leu Leu Leu Ile Thr Xaa
            260                 265

<210>  43
<211>  262
<212>  PRT
<213>  Artificial

<220>
<223>  putative protein sequence

<220>
<221>  misc_feature
<222>  (262)..(262)
<223>  Xaa can be any naturally occurring amino acid

<400>  43

Met Ala His Ala Ala Gln Val Gly Leu Gln Asp Ala Thr Ser Pro Ile
1               5                   10                  15

Met Glu Glu Leu Ile Thr Phe His Asp His Ala Leu Met Ile Ile Phe
            20                  25                  30

Leu Ile Cys Phe Leu Val Leu Tyr Ala Leu Phe Leu Thr Leu Thr Thr
        35                  40                  45

Lys Leu Thr Asn Thr Asn Ile Ser Asp Ala Gln Glu Met Glu Thr Val
    50                  55                  60

Trp Thr Ile Leu Pro Ala Ile Ile Leu Val Leu Ile Ala Leu Pro Ser
65                  70                  75                  80

Leu Arg Ile Leu Tyr Met Thr Asp Glu Val Asn Asp Pro Ser Leu Thr
                85                  90                  95

Ile Lys Ser Ile Gly His Gln Trp Tyr Trp Thr Tyr Glu Tyr Thr Asp
            100                 105                 110

Tyr Gly Gly Leu Ile Phe Asn Ser Tyr Met Leu Pro Pro Leu Phe Leu
        115                 120                 125

Glu Pro Gly Asp Leu Arg Leu Leu Asp Val Asp Asn Arg Val Val Leu
    130                 135                 140

Pro Ile Glu Ala Pro Ile Arg Met Met Ile Thr Ser Gln Asp Val Leu
145                 150                 155                 160

His Ser Trp Ala Val Pro Thr Leu Gly Leu Lys Thr Asp Ala Ile Pro
                165                 170                 175

Gly Arg Leu Asn Gln Thr Thr Phe Thr Ala Thr Arg Pro Gly Val Tyr
            180                 185                 190

Tyr Gly Gln Cys Ser Glu Ile Cys Gly Ala Asn His Ser Phe Met Pro
        195                 200                 205

Ile Val Leu Asp Leu Thr Trp Leu Glu Lys Leu Leu Pro Lys Thr Ile
    210                 215                 220

Ser Gln His Gln Ile Ser Thr Ser Ile Ile Thr Ser Thr Gln Lys Gly
225                 230                 235                 240

Met Ile Lys Leu Tyr Phe Leu Ser Phe Phe Phe Pro Leu Ile Leu Thr
                245                 250                 255

Leu Leu Leu Ile Thr Xaa
            260

<210>  44
<211>  43
<212>  PRT
<213>  Artificial

<220>
<223>  putative protein sequence


<220>
<221>  misc_feature
<222>  (43)..(43)
<223>  Xaa can be any naturally occurring amino acid

<400>  44

Met Pro Gln Leu Asn Thr Thr Val Trp Pro Thr Met Ile Thr Pro Met
1               5                   10                  15


Leu Leu Thr Leu Phe Leu Ile Thr Gln Leu Lys Met Leu Asn Thr Asn
            20                  25                  30


Tyr His Leu Pro Pro Ser Pro Leu Ala Ala Xaa
        35                  40


<210>  45
<211>  383
<212>  PRT
<213>  Artificial

<220>
<223>  putative protein sequence


<220>
<221>  misc_feature
<222>  (383)..(383)
<223>  Xaa can be any naturally occurring amino acid (or part thereof)

<400>  45

Met Leu Leu Ile Ser Ser Met Gln Pro Pro Pro Ile Leu Pro Ser Thr
1               5                   10                  15


His Thr Pro Leu Leu Thr Pro Tyr Pro Glu Asn Val Gly Tyr Thr Leu
            20                  25                  30


Pro Val Leu Ile Asn Pro Leu Ala Gln Pro Val Ile Tyr Ser Thr Ile
        35                  40                  45


Phe Ala Gly Thr Leu Ile Thr Ala Leu Ser Ser His Trp Phe Phe Thr
    50                  55                  60


Trp Val Gly Leu Glu Met Asn Met Leu Ala Phe Ile Pro Val Leu Thr
65                  70                  75                  80


Lys Lys Met Asn Pro Arg Ser Thr Glu Ala Ala Ile Lys Tyr Phe Leu
                85                  90                  95

Thr Gln Ala Thr Ala Ser Met Ile Leu Leu Met Ala Ile Leu Phe Asn
            100                 105                 110

Asn Met Leu Ser Gly Gln Trp Thr Met Thr Asn Thr Thr Asn Gln Tyr
        115                 120                 125

Ser Ser Leu Met Ile Met Met Ala Met Ala Met Lys Leu Gly Met Ala
    130                 135                 140

Pro Phe His Phe Trp Val Pro Glu Val Thr Gln Gly Thr Pro Leu Thr
145                 150                 155                 160

Ser Gly Leu Leu Leu Leu Thr Trp Gln Lys Leu Ala Pro Ile Ser Ile
                165                 170                 175

Met Tyr Gln Ile Ser Pro Ser Leu Asn Val Ser Leu Leu Leu Thr Leu
            180                 185                 190

Ser Ile Leu Ser Ile Met Ala Gly Ser Trp Gly Gly Leu Asn Gln Thr
        195                 200                 205

Gln Leu Arg Lys Ile Leu Ala Tyr Ser Ser Ile Thr His Met Gly Trp
    210                 215                 220

Met Met Ala Val Leu Pro Tyr Asn Pro Asn Met Thr Ile Leu Asn Leu
225                 230                 235                 240

Thr Ile Tyr Ile Ile Leu Thr Thr Thr Ala Phe Leu Leu Leu Asn Leu
                245                 250                 255

Asn Ser Ser Thr Thr Thr Leu Leu Leu Ser Arg Thr Trp Asn Lys Leu
            260                 265                 270

Thr Trp Leu Thr Pro Leu Ile Pro Ser Thr Leu Leu Ser Leu Gly Gly
        275                 280                 285

Leu Pro Pro Leu Thr Gly Phe Leu Pro Lys Trp Ala Ile Ile Glu Glu
    290                 295                 300

Phe Thr Lys Asn Asn Ser Leu Ile Ile Pro Thr Ile Met Ala Thr Ile
305                 310                 315                 320

Thr Leu Leu Asn Leu Tyr Phe Tyr Leu Arg Leu Ile Tyr Ser Thr Ser
                325                 330                 335

Ile Thr Leu Leu Pro Met Ser Asn Asn Val Lys Met Lys Trp Gln Phe
            340                 345                 350

Glu His Thr Lys Pro Thr Pro Phe Leu Pro Thr Leu Ile Ala Leu Thr
        355                 360                 365

Thr Leu Leu Leu Pro Ile Ser Pro Phe Met Leu Met Ile Leu Xaa
    370                 375                 380